# EUROPEAN PATENT APPLICATION

(11) **EP 4 800 668 A2**
(43) Date of publication of application: **02.09.2026**
(21) Application number: 26193268.5
(22) Date of filing: 30.07.2021
(51) Int. Cl.: G06V 40/20

(54) **AUTOMATED PHENOTYPING OF BEHAVIOR USING FEATURE VECTORS DETERMINED USING VIDEO DATA**

(30) Priority: 30.07.2020 US 202063058569 P
(62) Document number(s) of the earlier application(s) in accordance with Art. 76 EPC: 21762197.8 / 4 189 592
(71) Applicant: The Jackson Laboratory, Bar Harbor, ME 04609 (US)
(72) Inventor: KUMAR, Vivek, Bar Harbor, ME 04609 (US); WOTTON, Janine, Bar Harbor, ME 04609 (US); WHITE, Jacqui, Bar Harbor, ME 04609 (US)
(74) Representative: Barker Brettell LLP

(57) **Abstract**

Systems and methods described herein provide techniques for automated phenotyping. The systems and methods, in some embodiments, processes video data, identifies body parts, extracts features on a frame-level, and determines subject behavior captured in the video data. The systems and methods may use one or more machine learning models.

## Description

### Related Applications

This application claims benefit under 35 U.S.C. § 119(e) of U.S. Provisional application serial number 63/058,569, filed July 30, 2020, the disclosure of which is incorporated by reference herein in its entirety.

### Field of the Invention

The invention, in some aspects, relates to automated phenotyping of behavior, in particular, for nociception behaviors, by processing video data.

### Government Support

This invention was made with government support under R21DA048634 and UM1OD023222 awarded by the National Institutes of Health. The government has certain rights in the invention.

### Background

The phenomenon of pain is a complex combination of physical information, emotional context and personal subjective experience. Animal subjects are often used to study the neural and genetic bases of pain behaviors. Because the animal's subjective experience cannot be measured, other methods are developed to quantify "nociceptive" behaviors, which are defined as behavioral responses to painful stimuli. Many nociception assays depend on a quick motor withdrawal reflex in response to a brief mechanical or thermal stimulation and this simple movement is relatively easy to define and recognize, but such assays lack similarity to clinical pain. In mice, these assays are poorly correlated with more clinically relevant chronic pain assays and are more closely associated with startle and reactivity traits. A formalin assay was previously developed to assess nociceptive behaviors in rats and to monitor complex actions over an extended period in response to chemically induced, localized inflammation. Currently available formalin assays are limited of limited effectiveness by their reliance on human observers to identify when an animal exhibits nociceptive behavior, which makes the observation labor intensive, time consuming and subjective as the different nociceptive behaviors are not always uniformly defined and recorded.

### Summary of the Invention

According to an aspect of the invention, a computer-implemented method is provided, the method including: receiving video data representing a video capturing movements of a subject; determining, using the video data, first point data identifying a location of a first body part of the subject for a first frame during a first time period; determining, using the video data, second point data identifying a location of a second body part of the subject for the first frame; determining, using the first point data and the second point data, first distance data representing a distance between the first body part and the second body part; determining a first feature vector corresponding to at least the first frame and a second frame, the first feature vector including at least the first distance data and second distance data; processing, using a trained model, at least the first feature vector, the trained model configured to identify a likelihood of the subject exhibiting a behavior during the first time period; and determining, based on the processing of at least the first feature vector, a first label corresponding to the first time period, the first label identifying a first behavior of the subject during the first time period. In some embodiments, the method also includes determining, using the video data, third point data identifying a location of a third body part of the subject for the first frame. In certain embodiments, method also includes determining, using the first point data and the third point data, second distance data representing a distance between the first body part and the third body part; determining a second feature vector corresponding to the first frame to include at least the second distance data; and wherein processing using the trained model includes processing the first feature vector and the second feature vector using the trained model. In some embodiments, the method also includes determining, using the first point data, the second point data and the third point data, first angle data representing an angle corresponding to the first body part, the second body part and the third body part; determining a second feature vector corresponding to at least the first frame, the second feature vector including at least the first angle data; and wherein processing using the trained model also includes processing the first feature vector and the second feature vector using the trained model. In some embodiments, the method also includes determining, using the video data, fourth point data identifying a location of the first body part for a second frame during the first time period; determining, using the video data, fifth point data identifying a location of the second body part for the second frame; determining, using the video data, sixth point data identifying a location of the third body part for the second frame; determining, using the fourth point data and the fifth point data, third distance data representing a distance between the first body part and the second body part for the second frame; determining, using the fourth point data and the sixth point data, fourth distance data representing a distance between the first body part and the third body part for the second frame; determining, using the fourth point data, the fifth point data and the sixth point data, second angle data representing an angle corresponding to the first body part, the second body part and the third body part for the second frame; and determining the second feature vector to include at least the third distance data, the fourth distance data, and the second angle data. In certain embodiments, the second distance data represents a distance between the first body part and the second body part for the second frame during the first time period. In some embodiments, the method also includes calculating metric data corresponding to the first frame using at least the first distance data and the second distance data, wherein the first feature vector includes the metric data. In some embodiments, the metric data represents statistical analysis corresponding to at least the first distance data and the second distance data, the statistical analysis being at least one of a mean, a standard deviation, a median, and a median absolute deviation. In some embodiments, the method also includes: processing the video data using an additional trained model to determine the first point data, wherein the first point data includes pixel data representing the location of the first body part. In certain embodiments, the method also includes processing the video data using an additional trained model to determine a likelihood that a pixel coordinate corresponds to the first body part; and determining the first point data based at least in part on the likelihood satisfying a threshold, the first point data including the pixel coordinate. In certain embodiments, the method also includes determining, using the video data, additional point data identifying locations of at least 12 portions of the subject for the first frame, wherein the 12 portions includes at least the first body part and the second body part. In some embodiments, the method also includes determining additional distance data representing distances between a plurality of body portion-pairs for the first frame, the plurality of body portion-pairs formed using pairs of the 12 portions of the subject, wherein the first feature vector includes the additional distance data. In some embodiments, the method also includes determining additional angle data representing angles corresponding to a plurality of body-portion trios for the first frame, the plurality of body portion-trios formed by selecting three of the 12 portions of the subject, and wherein the first feature vector includes the additional angle data. In some embodiments, the method also includes: determining additional feature vectors corresponding to six frames during the first time period, the six frames including at least the first frame and the second frame; calculating metric data using the additional feature vectors, the metric data representing at least one of a mean, a standard deviation, a median, and a median absolute deviation; and processing the metric data using the trained model to determine the first label. In certain embodiments, the method also includes determining location data representing pixel coordinates of 12 portions of the subject for the first frame, the location data including at least the first point data, the second point data and the third point data, and wherein processing the metric data using the trained model further includes processing the location data using the trained model. In some embodiments, the method also includes determining additional feature vectors corresponding to 11 frames during the first time period, the 11 frames including at least the first frame and the second frame; calculating metric data using the additional feature vectors, the metric data representing at least one of a mean, a standard deviation, a median, and a median absolute deviation; and processing the metric data using the trained model to determine the first label. In certain embodiments, the method also includes the 11 frames includes five frames prior to the first frame and five frames after the first frame. In certain embodiments, the method also includes determining additional feature vectors corresponding to 21 frames during the first time period, the 21 frames including at least the first frame and the second frame; calculating metric data using the additional feature vectors, the metric data representing at least one of a mean, a standard deviation, a median, and a median absolute deviation; and processing the metric data using the trained model to determine the first label. In some embodiments, the 21 frames includes 11 frames prior to the first frame and 11 frames after the first frame. In some embodiments, the video data represents video capturing movements of more than one subject. In some embodiments, receiving video data comprises receiving video data obtained from a top-down video of the subject. In some embodiments, receiving video data comprises receiving video data that is not obtained from a top-down video of the subject. In some embodiments, the video comprises a top-down video. In some embodiments, the video comprises other than a top-down video. In certain embodiments the video does not comprise a top-down video. In certain embodiments, the trained model is a classifier configured to process feature data corresponding to video frames to determine a behavior exhibited by the subject represented in the video frames, the feature data corresponding to portions of the subject. In certain embodiments, the first body part is a mouth of the subject; the second body part is right hind foot of the subject; the trained model is configured to identify a likelihood of the subject exhibiting contact between the first body part and the second body part; and the first label indicates the first frame represents contact between the first body part and the second body part. In some embodiments, the first frame corresponds to 30 milliseconds of video data. In certain embodiments, the video data corresponds to a first video capturing a top view of the subject and a second video capturing a side view of the subject. In some embodiments, the subject is a mammal. In some embodiments, the subject is a rodent. In some embodiments, the subject is a primate. In certain embodiments, the subject is a genetically engineered subject. In certain embodiments, the subject is a mouse. In some embodiments, the mouse is a genetically engineered rodent. In certain embodiments the subject is a genetically engineered mouse.

According to another aspect of the invention, a method of determining a nociceptive behavior in a test subject is provided, the method including monitoring a response of the test subject, wherein a means of the monitoring includes a computer-implemented method of any embodiment of any of the aforementioned methods. In certain embodiments, the test subject has a pain condition. In some embodiments, the pain condition includes one of more of: inflammatory pain, neuropathic pain, muscle pain, joint pain, chronic pain, visceral pain, cancer pain, and postoperative pain. In some embodiments, the test subject is an animal model of a pain condition. In certain embodiments, a pain is induced in the test subject. In some embodiments, inducing the pain includes contacting the test subject with one or more of: heat, light, pressure, cold, and a chemical agent. In some embodiments, a means of inducing the pain in the test subject includes inducing inflammation in the test subject. In some embodiments, a means of inducing the inflammation includes contacting the test subject with one or more of: heat, light, pressure, cold, and a chemical agent. In certain embodiments, the chemical agent includes one or more of formalin and acetone. In certain embodiments, a means of inducing the pain includes one or more of contacting the test subject with the noxious stimuli, for example a pain-inducing chemical agent, implanting a noxious-stimuli-generating element into the subject, injecting the test subject with the chemical agent that induces pain, etc. In some embodiments, the test subject is a genetically engineered test subject. In certain embodiments, the test subject is a rodent, and optionally is a mouse. In some embodiments, the test subject is a genetically engineered rodent. In certain embodiments the test subject is a genetically engineered mouse. In some embodiments, the method also includes administering a candidate therapeutic agent to the test subject. In some embodiments, if pain is induced in the test subject, the candidate therapeutic agent is administered to the test subject prior to inducing the pain in the test subject. In certain embodiments, if pain is induced in the test subject, the candidate therapeutic agent is administered to the test subject at one of more of during and after the pain induction in the test subject. In certain embodiments, the candidate agent is administered to the test subject one time. In some embodiments, the candidate therapeutic agent is administered to the test subject two, three, four, five, or more times. In certain embodiments, the two or more times the candidate therapeutic agent is administered to the test subject are all before, all during, or all after the pain induction. In some embodiments, the two or more times the candidate therapeutic agent is administered to the test subject are a combination of two or more of before, during, and after the pain induction. In some embodiments, a result of the monitoring of the test subject is compared to a control result. In certain embodiments, the control result is a result from a control subject monitored with the computer-implemented method. In certain embodiments, pain is induced in the control subject. In some embodiments, the pain induced in the control subject is substantially equivalent to the pain induced in the test subject. In certain embodiments, the control subject is an animal model of the pain condition. In some embodiments, the test animal and the control animal are both animal models of the pain condition. In some embodiments, the test and control animal models are the same animal models. In certain embodiments the test and control animal models are different animal models. In some embodiments, the control subject is not administered the candidate therapeutic agent. In some embodiments, the control subject is administered an amount (also referred to as "a dose") of the candidate therapeutic agent that is different than the amount (dose) of the candidate therapeutic agent administered to the test subject. In certain embodiments, the control result is a result from a previous monitoring of the test subject with the computer-implemented method. In certain embodiments, the monitoring of the subject identifies a chronic pain condition in the subject. In certain embodiments, the monitoring of the subject identifies efficacy of a candidate therapeutic agent to treat a pain condition.

According to another aspect of the invention, a method of identifying efficacy of a candidate therapeutic agent to treat a pain condition in a subject is provided, the method including: administering to a test subject the candidate therapeutic agent and monitoring the test subject, wherein a means of the monitoring includes a computer-implemented method of any embodiment of any of the aforementioned aspects of the invention, and wherein results of the monitoring indicating reduced pain in the test subject identifies an efficacy of the candidate therapeutic agent to treat the pain condition. In some embodiments, the pain condition includes one of more of: inflammatory pain, neuropathic pain, muscle pain, joint pain, chronic pain, visceral pain, cancer pain, and postoperative pain. In some embodiments, the test subject has the pain condition. In certain embodiments, the test subject is an animal model of the pain condition. In certain embodiments, a pain is induced in the test subject prior to the monitoring. In some embodiments, inducing the pain in the test subject includes inducing inflammation in the test subject. In certain embodiments, inducing inflammation includes contacting the test subject with one or more of: heat, light, pressure, cold, and a chemical agent. In certain embodiments, the chemical agent includes one or more of formalin and acetone, and wherein inducing the pain includes one or more of contacting the test subject with the chemical agent, inserting the chemical agent into the test subject, implanting the chemical agent into the test subject, and injecting the test subject with the chemical agent. In certain embodiments, a means of inducing pain in a subject includes one or more of contacting the subject with an effective amount of a pain-inducing agent. In some embodiments, a means of inducing pain in a subject includes one or more of: contacting subject with an effective amount of a pain-inducing chemical agent; implanting a pain-inducing agent, such as but not limited to a stimulating electrode, a slow-release chemical agent, etc. into or onto the subject; injecting the subject with a chemical agent that induces pain, etc. In some embodiments, the test subject is a genetically engineered test subject. In some embodiments, the test subject is a rodent, and optionally is a mouse. In certain embodiments, the test subject is a genetically engineered rodent. In some embodiments the test subject is a genetically engineered mouse. In some embodiments, the candidate therapeutic agent is administered to the test subject prior to inducing the pain in the test subject. In certain embodiments, the candidate therapeutic agent is administered to the test subject at one of more of: during and after the pain induction in the test subject. In some embodiments, the candidate agent is administered to the test subject one time. In some embodiments, the candidate therapeutic agent is administered to the test subject two, three, four, five, or more times. In certain embodiments, the two or more times the candidate therapeutic agent is administered to the test subject are all before, all during, or all after the pain induction. In certain embodiments, the two or more times the candidate therapeutic agent is administered to the test subject are a combination of two or more of before, during, and after the pain induction. In some embodiments, a result of the monitoring of the test subject is compared to a control result. In some embodiments, the control result is a result from a control subject monitored with the computer-implemented method. In certain embodiments, pain is induced in the control subject. In some embodiments, the pain induced in the control subject is substantially equivalent to the pain induced in the test subject. In certain embodiments, the control subject has the pain condition, and optionally is an animal model of the pain condition. In some embodiments, the test animal and the control animal are both animal models of the pain condition. In some embodiments, the control subject is not administered the candidate therapeutic agent. In certain embodiments, the test and control animal models are the same animal models. In some embodiments the test and control animal models are different animal models. In some embodiments, the control subject is administered an amount (also referred to as "a dose") of the candidate therapeutic agent that is different than the amount or dose respectively of the candidate therapeutic agent administered to the test subject. In certain embodiments, the control result is a result from a previous monitoring of the test subject with the computer-implemented method. In some embodiments, the method also includes additionally testing the efficacy of the candidate therapeutic agent.

According to another aspect of the invention, a system is provided, the system including: at least one processor; and at least one memory including instructions that, when executed by the at least one processor, cause the system to: receive video data representing a video capturing movements of a subject; determine, using the video data, first point data identifying a location of a first body part of the subject for a first frame during a first time period; determine, using the video data, second point data identifying a location of a second body part of the subject for the first frame; determine, using the first point data and the second point data, first distance data representing a distance between the first body part and the second body part; determine a first feature vector corresponding to at least the first frame and a second frame, the first feature vector including at least the first distance data and second distance data; process, using a trained model, at least the first feature vector, the trained model configured to identify a likelihood of the subject exhibiting a behavior during the first time period; and determine, based on the processing of at least the first feature vector, a first label corresponding to the first time period, the first label identifying a first behavior of the subject during the first time period. In some embodiments, the at least one memory also includes instructions that, when executed by the at least one processor, further cause the system to: determine, using the video data, third point data identifying a location of a third body part of the subject for the first frame. In certain embodiments, the at least one memory also includes instructions that, when executed by the at least one processor, further cause the system to: determine, using the first point data and the third point data, second distance data representing a distance between the first body part and the third body part; determine a second feature vector corresponding to the first frame to include at least the second distance data; and wherein the instructions that cause the system to process using the trained model further causes the system to process the first feature vector and the second feature vector using the trained model. In certain embodiments, the at least one memory also includes instructions that, when executed by the at least one processor, further cause the system to: determine, using the first point data, the second point data and the third point data, first angle data representing an angle corresponding to the first body part, the second body part and the third body part; determine a second feature vector corresponding to at least the first frame, the second feature vector including at least the first angle data; and wherein the instructions that cause the system to process using the trained model further causes the system to process the first feature vector and the second feature vector using the trained model. In certain embodiments, the at least one memory also includes instructions that, when executed by the at least one processor, further cause the system to: determine, using the video data, fourth point data identifying a location of the first body part for a second frame during the first time period; determine, using the video data, fifth point data identifying a location of the second body part for the second frame; determine, using the video data, sixth point data identifying a location of the third body part for the second frame; determine, using the fourth point data and the fifth point data, third distance data representing a distance between the first body part and the second body part for the second frame; determine, using the fourth point data and the sixth point data, fourth distance data representing a distance between the first body part and the third body part for the second frame; determine, using the fourth point data, the fifth point data and the sixth point data, second angle data representing an angle corresponding to the first body part, the second body part and the third body part for the second frame; and determine the second feature vector to include at least the third distance data, the fourth distance data, and the second angle data. In some embodiments, the second distance data represents a distance between the first body part and the second body part for the second frame during the first time period. In certain embodiments, the at least one memory also includes instructions that, when executed by the at least one processor, further cause the system to: calculate metric data corresponding to the first frame using at least the first distance data and the second distance data, wherein the first feature vector includes the metric data. In some embodiments, the metric data represents statistical analysis corresponding to at least the first distance data and the second distance data, the statistical analysis being at least one of a mean, a standard deviation, a median, and a median absolute deviation. In some embodiments, the at least one memory also includes instructions that, when executed by the at least one processor, further cause the system to: process the video data using an additional trained model to determine the first point data, wherein the first point data includes pixel data representing the location of the first body part. In some embodiments, the at least one memory also includes instructions that, when executed by the at least one processor, further cause the system to: process the video data using an additional trained model to determine a likelihood that a pixel coordinate corresponds to the first body part; and determine the first point data based at least in part on the likelihood satisfying a threshold, the first point data including the pixel coordinate. In certain embodiments, the at least one memory also includes instructions that, when executed by the at least one processor, further cause the system to: determine, using the video data, additional point data identifying locations of at least 12 portions of the subject for the first frame, wherein the 12 portions includes at least the first body part and the second body part. In certain embodiments, the at least one memory also includes instructions that, when executed by the at least one processor, further cause the system to: determine additional distance data representing distances between a plurality of body portion-pairs for the first frame, the plurality of body portion-pairs formed using pairs of the 12 portions of the subject, and wherein the first feature vector includes the additional distance data. In certain embodiments, the at least one memory also includes instructions that, when executed by the at least one processor, further cause the system to: determine additional angle data representing angles corresponding to a plurality of body-portion trios for the first frame, the plurality of body portion-trios formed by selecting three of the 12 portions of the subject, and wherein the first feature vector includes the additional angle data. In some embodiments, the at least one memory also includes instructions that, when executed by the at least one processor, further cause the system to: determine additional feature vectors corresponding to six frames during the first time period, the six frames including at least the first frame and the second frame; calculate metric data using the additional feature vectors, the metric data representing at least one of a mean, a standard deviation, a median, and a median absolute deviation; and process the metric data using the trained model to determine the first label. In some embodiments, the at least one memory also includes instructions that, when executed by the at least one processor, further cause the system to: determine location data representing pixel coordinates of 12 portions of the subject for the first frame, the location data including at least the first point data, the second point data and the third point data, and wherein the instructions that cause the system to process the metric data using the trained model further causes the system to process the location data using the trained model. In certain embodiments, the at least one memory also includes instructions that, when executed by the at least one processor, further cause the system to: determine additional feature vectors corresponding to 11 frames during the first time period, the 11 frames including at least the first frame and the second frame; calculate metric data using the additional feature vectors, the metric data representing at least one of a mean, a standard deviation, a median, and a median absolute deviation; and process the metric data using the trained model to determine the first label. In certain embodiments, the 11 frames includes five frames prior to the first frame and five frames after the first frame. In some embodiments, the at least one memory also includes instructions that, when executed by the at least one processor, further cause the system to: determine additional feature vectors corresponding to 21 frames during the first time period, the 21 frames including at least the first frame and the second frame; calculate metric data using the additional feature vectors, the metric data representing at least one of a mean, a standard deviation, a median, and a median absolute deviation; and process the metric data using the trained model to determine the first label. In certain embodiments, the 21 frames includes 11 frames prior to the first frame and 11 frames after the first frame. In certain embodiments, the video data represents video capturing movements of more than one subject. In some embodiments, the trained model is a classifier configured to process feature data corresponding to video frames to determine a behavior exhibited by the subject represented in the video frames, the feature data corresponding to portions of the subject. In some embodiments, the first body part is a mouth of the subject; the second body part is right hind foot of the subject; the trained model is configured to identify a likelihood of the subject exhibiting contact between the first body part and the second body part; and the first label indicates the first frame represents contact between the first body part and the second body part. In some embodiments, the first frame corresponds to 30 milliseconds of video data. In certain embodiments, the video data corresponds to a first video capturing a top view of the subject and a second video capturing a side view of the subject. In some embodiments, video data comprises video data obtained from a top-down video of the subject. In some embodiments, video data comprises video data that is side-view video of the subject. In certain embodiments, the subject is a mammal. In certain embodiments, the subject is a rodent. In some embodiments, the subject is a primate. In certain embodiments, the subject is a genetically engineered subject. In certain embodiments, the subject is a rodent, and optionally is a mouse. In some embodiments, the subject is a genetically engineered rodent. In some embodiments the subject is a genetically engineered mouse.

According to another aspect of the invention, one or more non-transitory computer-readable media are provided, and include computer executable instructions that, when executed, cause at least one processor to perform actions including: receiving video data representing a video capturing movements of a subject; determining, using the video data, first point data identifying a location of a first body part of the subject for a first frame during a first time period; determining, using the video data, second point data identifying a location of a second body part of the subject for the first frame; determining, using the first point data and the second point data, first distance data representing a distance between the first body part and the second body part; determining a first feature vector corresponding to at least the first frame and a second frame, the first feature vector including at least the first distance data and second distance data; processing, using a trained model, at least the first feature vector, the trained model configured to identify a likelihood of the subject exhibiting a behavior during the first time period; and determining, based on the processing of at least the first feature vector, a first label corresponding to the first time period, the first label identifying a first behavior of the subject during the first time period. In some embodiments, receiving video data comprises receiving video data obtained from one or both of a top-down video of the subject and a side-view video of the subject. In some embodiments, receiving video data comprises receiving video data not obtained from a top-down video of the subject. In certain embodiments, the subject is a genetically engineered test subject. In some embodiments, the subject is a rodent, and optionally is a mouse. In some embodiments, the subject is a genetically engineered rodent. In certain embodiments the subject is a genetically engineered mouse.

### Brief Description of the Drawings

For a more complete understanding of the present disclosure, reference is now made to the following description taken in conjunction with the accompanying drawings.
FIG. 1 is a conceptual diagram of a system for determining subject behavior, according to embodiments of the present disclosure.
FIG. 2 is a flowchart illustrating a process for analyzing video data of a subject(s) to determine subject behavior, according to embodiments of the present disclosure.
FIG. 3 shows an example image of the video analyzed by the system to determine subject behavior.
FIG. 4A is a conceptual diagram of a subject mouse with various body parts marked for detection by the system, according to embodiments of the present disclosure.
FIG. 4B is a conceptual diagram illustrating the various body parts of a subject mouse detected by the system, according to embodiments of the present disclosure.
FIGS. 5 and 6 are flowcharts illustrating processes for generating feature vectors, according to embodiments of the present disclosure.
FIG. 7A is a conceptual diagram illustrating distance data representing the distance between two body parts of a subject during multiple video frames, according to embodiments of the present disclosure.
FIG. 7B is a conceptual diagram illustrating angle data representing the angle between three body parts of a subject during multiple video frames, according to embodiments of the present disclosure.
FIG. 8 is a flowchart illustrating a process for classifying subject behavior, according to embodiments of the present disclosure.
FIG. 9 illustrates an annotated image of the video analyzed by the system for subject behavior including system-annotations and human-annotations identifying subject body parts.
FIG. 10 illustrates a table showing contribution of the various window sizes for different body parts used by the system to analyze the video data, according to embodiments of present disclosure.
FIGS. 11A and 11B show graphs of the system determining subject behavior and a human annotator identifying subject behavior for each second of two short videos. In FIGS. 11A and 11B results for "Lick" and "No Lick" indicate results of the model with crosshatching and the human results as solid. In FIG. 11B, in the two vertical pairs of + signs, the top + top is for model results and the lower + is for human results. NL is No Lick.
FIG. 12 shows graphs of the percentage of agreement between the system determined subject behavior and the human annotator identified subject behavior for multiple videos including four subjects in four arenas.
FIG. 13 shows graphs comparing manual labels by two human annotators with the system determined labels for three mice subjects. Dots represent system determined labels; squares represent manual labels by human annotator "Observer 1"; and triangles represent manual labels by human annotator "Observer 2".

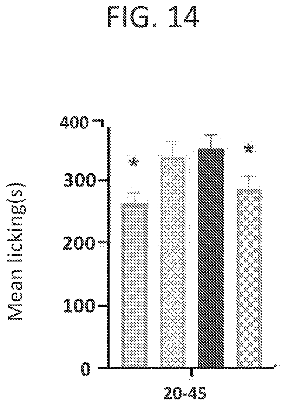

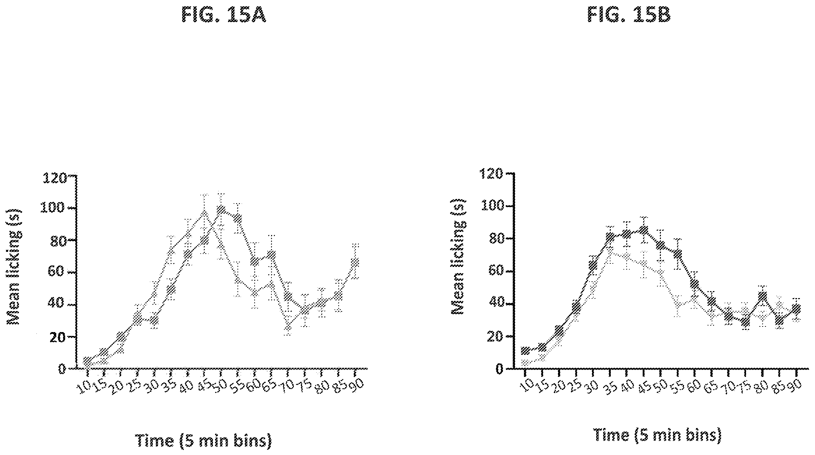

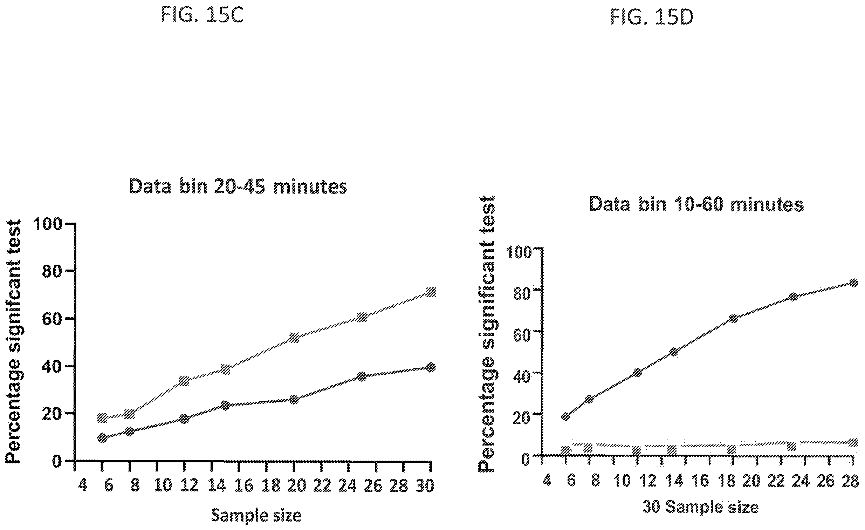

FIGS. 14 and 15A-D show graphs comparing licking behavior in male and female mice subjects of different strains. In FIG. 14, the solid bar on the left side of the graph represents female C57BL/6J mice; the small-checked bar on the left side of the graph represents female C57BL/6NJ mice; the solid bar on the right side of the graph represents male C57BL/6J mice; and the large-checked bar on the right side of the graph represents male C57BL/6NJ mice. In FIG. 15A, squares represent female C57BL/6J mice and triangles represent female C57BL/6NJ mice. In FIG. 15B, squares represent male C57BL/6J mice and inverted triangles represent male C57BL/6NJ mice. In FIG. 15C and 15D, squares represent females and dots represent males.
FIG. 16 is a block diagram conceptually illustrating example components of a device according to embodiments of the present disclosure.
FIG. 17 is a block diagram conceptually illustrating example components of a server according to embodiments of the present disclosure.

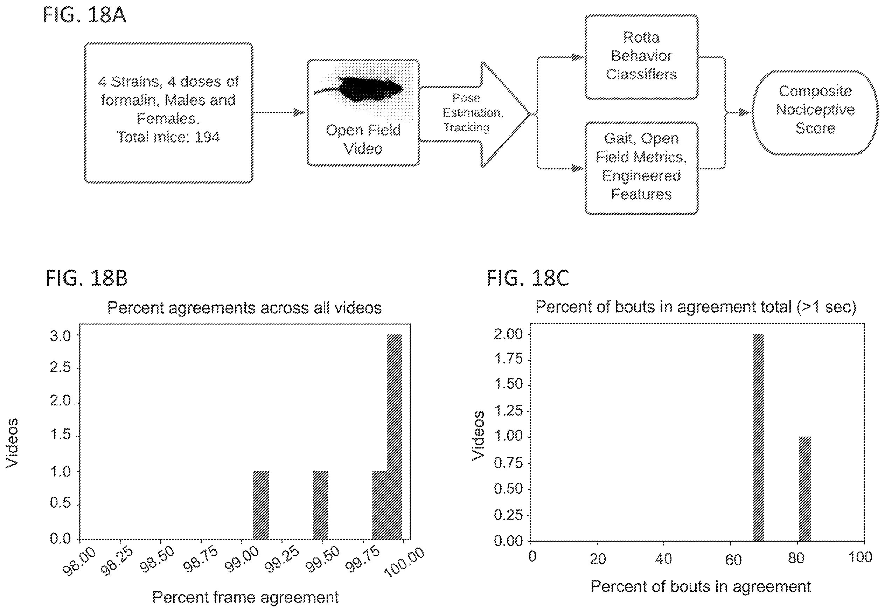

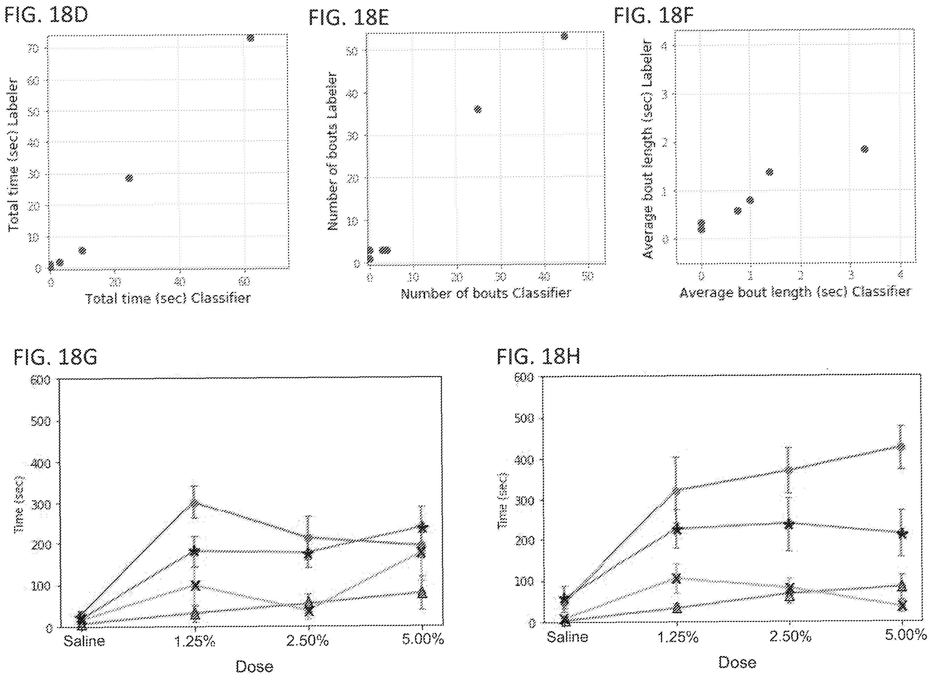

FIG. 18A shows a conceptual diagram illustrating development and testing of a composite nociceptive score according to embodiments of the present disclosure. FIG. 18B-18H shows graphs illustrating development and testing of a composite nociceptive score. FIGS. 18B-18F show graphs illustrating measurements of agreement between classifier and human scoring (Labeler). FIGS. 18G-18H show graphs illustrating dose-based and strain-based comparisons of time spent licking by male (FIG. 18G) and female (FIG. 18H) mice; dots represent strain C57BL6J, triangles represent strain AJ, "X"s represent strain BALBcJ, and asterisks represent strain C3HHeJ.

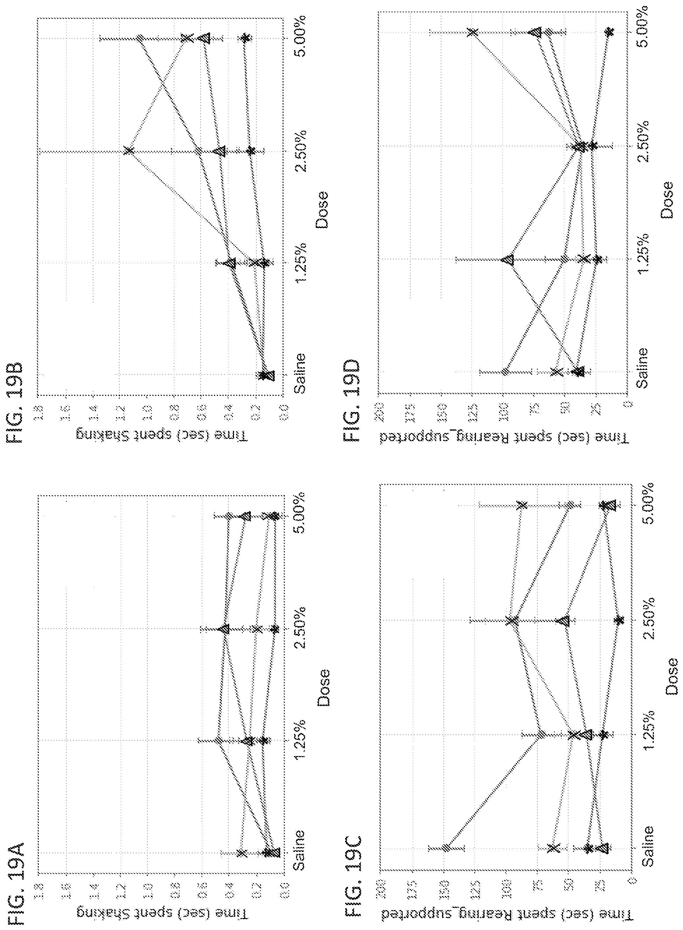

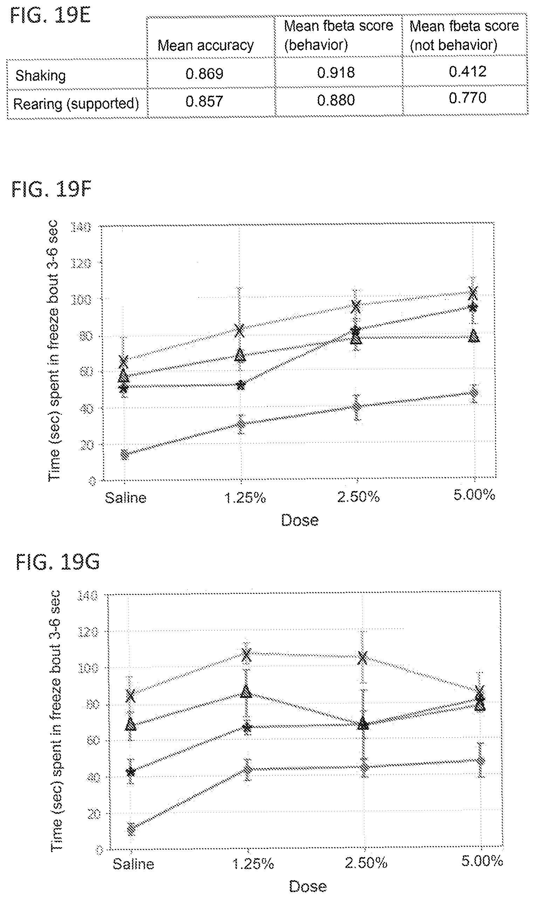

FIG. 19A-G shows graphs and a chart illustrating measurements of time spent paw shaking, rearing, and in freezing bouts, comparing results in male and female mice from strains C57BL6/J, AJ, BALBcJ, and C3HHeJ. FIG. 19A and 19B shows time male mice (FIG. 19A) and female mice (FIG. 19B) spent paw shaking by strain. FIG. 19C and 19D shows time male mice (FIG. 19C) and female mice (FIG. 19D) spent rearing by strain. The table of FIG. 19E shows accuracy measurements. FIG. 19F and 19G shows time male (FIG. 19F) and female (FIG. 19G) spent in freezing bouts of 3-6 seconds. In each of FIG. 19A-19D and FIG. 19F-19G, dots represent strain C57BL6J, triangles represent strain AJ, "X"s represent strain BALBcJ, and asterisks represent strain C3HHeJ.

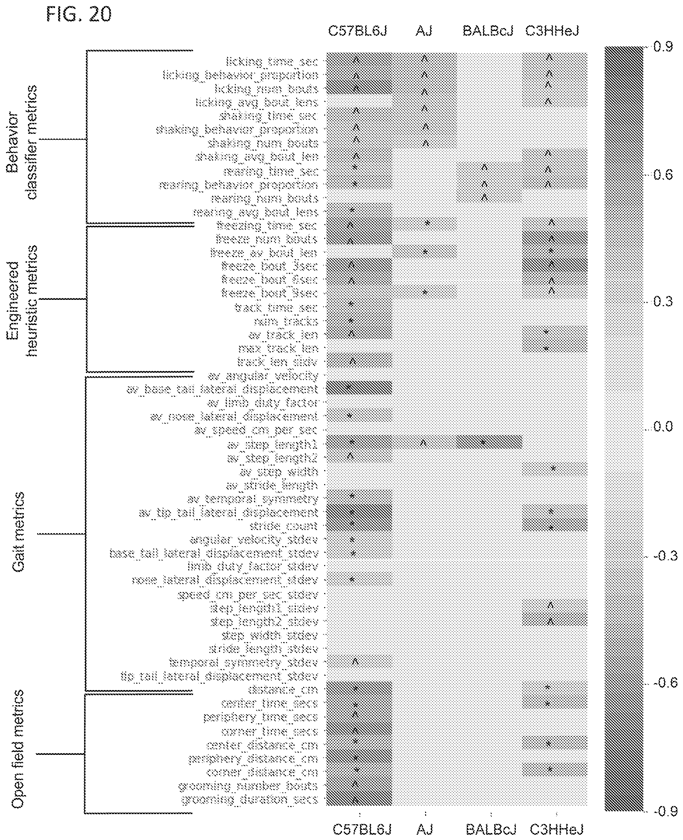

FIG. 20 shows a heatmap graph illustrating strain differences across all metrics demonstrating correlations of each metric with formalin dose across the four mouse strains. Metrics are categorized into four types: (1) behavior classifier metrics from the JAX Animal Behavior al System (JABS) system, [Kabra, M.. et al., 2013 Nature Methods Jan;10(1):64-7, the content of which is incorporated herein by reference in its entirety]; (2) engineered heuristic metrics; (3) gait metrics derived from a deep neural network; and (4) classical open field metrics derived from tracking and classification. The intensity of the colors corresponds to the magnitude of the correlation coefficient between the metric and dose as either positive (^) or negative (*). Only statistically significant correlations are shown.

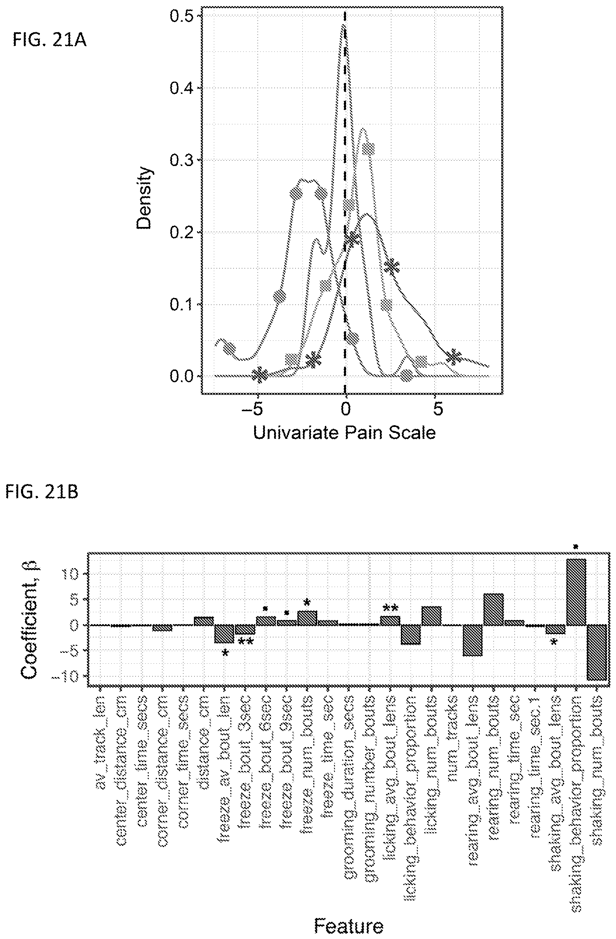

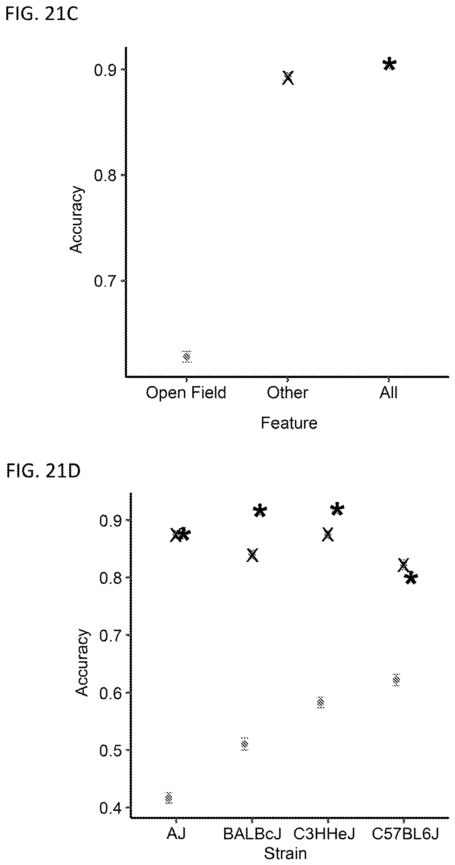

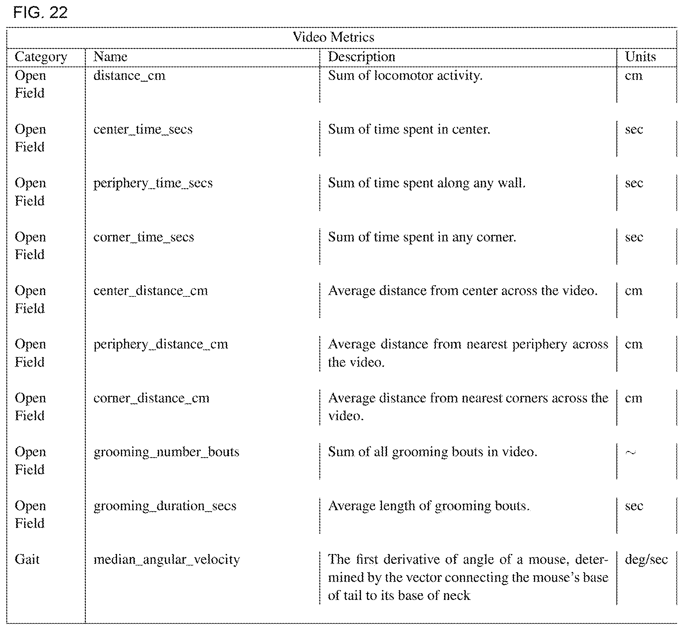

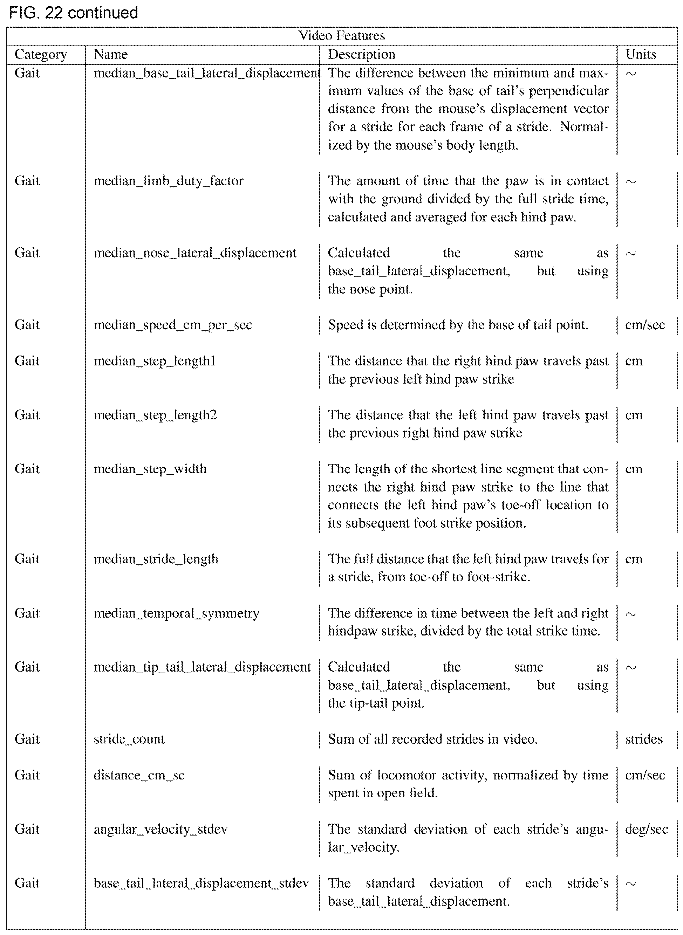

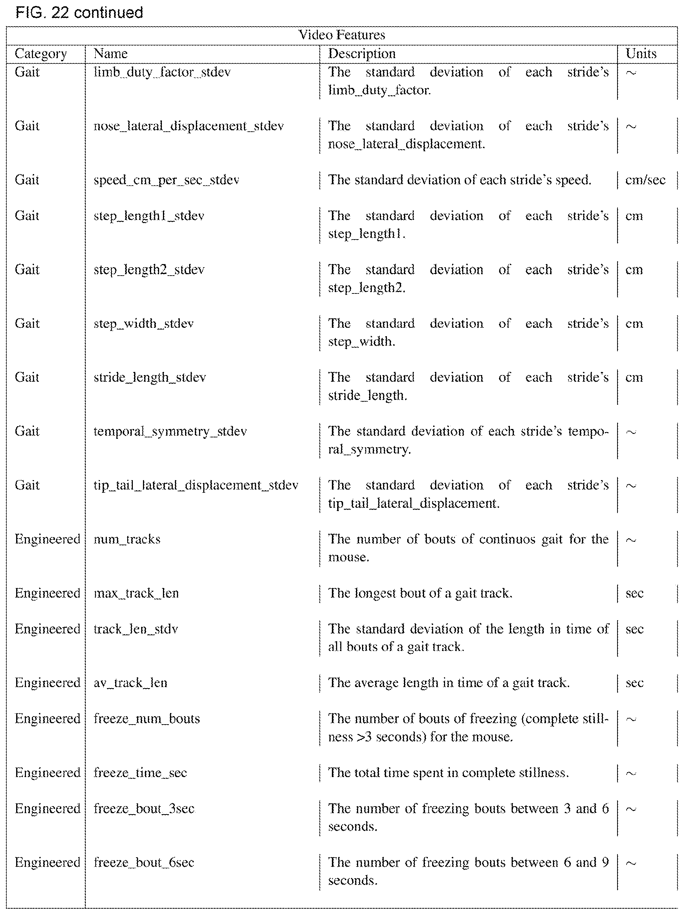

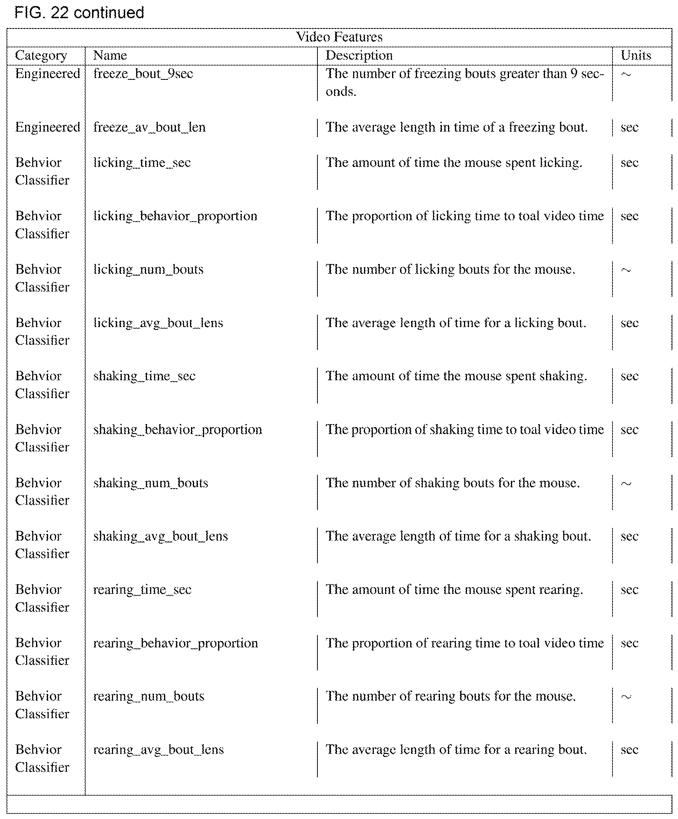

FIG. 21A-D presents graphs illustrating building of a univariate pain scale and cross-validation assessments of a binary logistic regression model. FIG. 21A shows a line graph illustrating a cumulative link model (logit link) fitted to the ordinal response (Dose) using a subset of features in FIG. 22. Solid dots indicate the line plotting Dose level 0; a solid gray line indicates the line plotting Dose level 1; solid squares indicate the line plotting Dose level 2; and asterisks indicate the line plotting Dose level 3. FIG. 21B shows a bar graph illustrating the contributions of individual features to the univariate pain scale. The significance of each feature's contribution is denoted using ** for highly significant, * for significant, and ▪ for suggestive contributions. FIG. 21C shows a graph illustrating leave one animal out cross-validation assessment of the accuracy metric of a binary logistic regression model built using different sets of features ("Open field" (solid gray dot); "Other", including engineered features and features obtained from behavior classifier ("X"); and "All", including both Open field and Other ("*"). FIG. 21D shows a graph illustrating leave one strain out cross-validation assessment of the accuracy metric of a binary logistic regression model built using different sets of features ("Open field" (solid gray dot); "Other", including engineered features and features obtained from behavior classifier ("X"); and "All", including both Open field and Other ("*"). The error bars in both FIG. 21C and FIG. 21D denote parametric bootstrap confidence intervals.
FIG. 22 provides information on video features of various behaviors observed in certain embodiments of methods of the invention.

### Detailed Description

The invention includes, in part, a method utilizing video recordings and machine learning techniques comprising three components; key point detection, per frame feature extraction using these key points, and algorithmic classification of behavior. This approach to automation is flexible as different model classifiers or key points can be used with only small losses in accuracy. Methods and systems of the invention comprise a machine learning scoring system and provides the required accuracy, consistency and ease of use that permits use of a noxious stimuli-based system, a non-limiting example of which is a formalin assay, a feasible choice for large-scale genetic studies. Methods and systems of the invention provide a reliable and scalable automated scoring system for nociception behavior and dramatically lowers time and labor costs associated with nociception experiments and also reduces variability in such experiments.

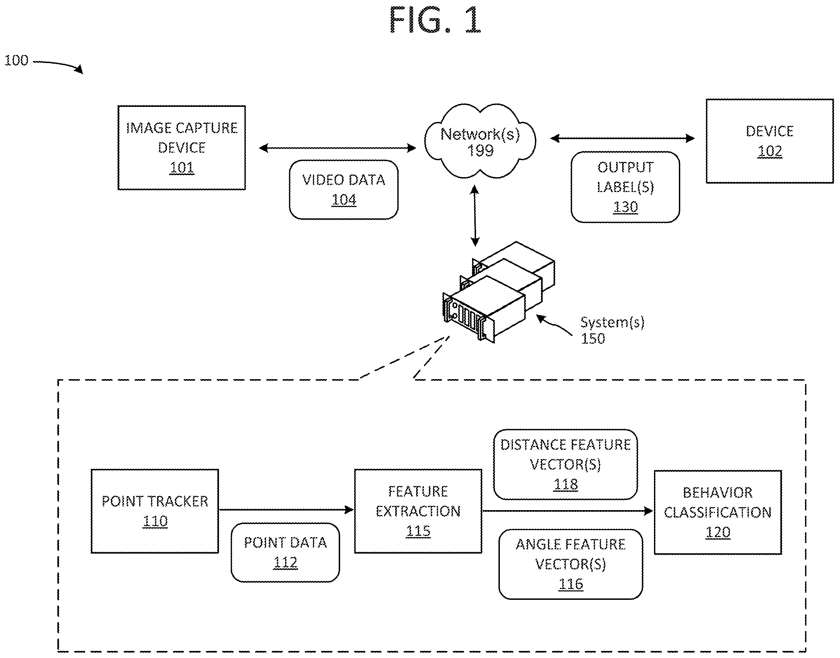

Some aspects of methods and systems of the invention comprise automated measurement of licking in the widely used open field arena with a top-down camera view. Certain embodiments of methods and systems of the invention comprise automated measurement of a variety of possible nocifensive behaviors, which are used for the purpose of a composite nociceptive index. Methods of the invention can be used for efficient quantification of multiple nocifensive behaviors in a video. In certain embodiments of methods and systems of the invention, top-down video of each mouse in a one hour open field session was collected according to previously published protocols (Fig. 1A). [see Kumar, V. et al., PNAS 108, 15557-15564, (2011) and Geuther, B. et al., Communications Biology 2, 124 (Mar. 2019), the content of each is incorporated by reference herein in its entirety]. The open field video was processed by a deep neural network based pose estimation network and a tracking network to produce a 12-point pose skeleton and an ellipse fit track of the mouse for each frame [Sheppard, K. et al., bioRxiv 424780 (2020) and Geuther, B. et al., Communications Biology 2, 124 (Mar. 2019), the content of each is incorporated herein in its entirety]. These per-frame measures were used to make behavior classifiers using JABS. The per-frame measures were also used to engineer features such as traditional open field measures of anxiety, hyperactivity, neural network-based grooming, and novel gait measures. All features are defined in Supplementary Figure 22. Embodiments of methods and systems of the invention can also be used to assess genetic variation in nociceptive response. As a non-limiting example, male and female mice selected from strains ranging from known higher licking responders (C57BL6J and C3HHeJ) to lower licking responders (BALBcJ and AJ) and examined using methods and systems of the invention and the results compared.

The phenomenon of pain is a complex combination of physical information, emotional context and personal subjective experience. Animal subjects are often used to study the neural and genetic bases of pain behaviors. Because the animal's subjective experience cannot be measured, other methods are developed to quantify "nociceptive" behaviors, which are defined as behavioral responses to painful stimuli, and "nocifensive" behaviors, which are defined as behaviors associated with protection against insult and injury. Many nociception assays depend on a quick motor withdrawal reflex in response to a brief mechanical or thermal stimulation and this simple movement is relatively easy to define and recognize, but such assays lack similarity to clinical pain. In mice, these assays are poorly correlated with more clinically relevant chronic pain assays and are more closely associated with startle and reactivity traits. In contrast, the formalin test, originally developed for use with rats, was designed to monitor complex actions over an extended period, in response to chemically induced, localized inflammation. The irritant formalin is usually injected in one hind paw and then the animal is observed for nociceptive behaviors such as licking, biting, lifting, flicking, shaking, or clutching of the injected paw. Formalin typically produces a biphasic response, with a short intense acute reaction (Phase I) from 0 - 10 minutes post-injection, a brief interphase of low response and then a sustained (Phase II) response, starting at about 10-15 minutes post-injection, increasing to a peak and then gradually subsiding, with an elevated response often still maintained at 60 minutes or more post-injection. This assay is a validated form of non-stimulus evoked spontaneous nociceptive behavior and the sustained nature of the behaviors are particularly pertinent to biological understanding of chronic pain. "Nocifensive" behaviors including paw shaking, favoring, and locomotor activity, are more complex than nociceptive behaviors and may be included in composite scores, though are also difficult to manually score in mice.

The formalin assay relies on human observers to identify when an animal exhibits nociceptive behavior, which makes the observation labor intensive, time consuming and subjective as the different nociceptive behaviors are not always uniformly defined and recorded. Rating scales are subject to inter-observer variability and some behaviors, such as favoring or lifting, are reportedly hard to score reliably in mice. Consequently, labeling of mouse behaviors are often restricted to licking/biting behaviors.

The present disclosure relates to automated phenotyping of subject behaviors, in particular, for formalin assays. Automated phenotyping of behaviors may overcome the foregoing scoring biases and inefficiencies related to human observers. The formalin assays can be recorded via video, and the video may be processed using the systems and methods described herein to label subject behaviors.

The systems and methods of the present disclosure may be particularly useful in phenotyping behaviors of smaller subjects, such as mice, and may be capable of distinguishing between smaller or similar movements, such as biting or licking. Smaller subjects, like mice, tend to move quickly compared to other larger subjects (like rats, etc.). The present disclosure describes techniques that can assess specific behaviors in subjects using high-speed video, and accurately assess rapid withdrawal reflex action used in many nociception assays.

Conventional systems track one or two body parts of a subject and also use physical markers on the subject (such as fur bleaching or using dyes on the subject). The present disclosure involves tracking multiple points on the subject body to measure changing distances between various body parts to determine when the subject exhibits nociceptive behavior. Subject behaviors are instantiated as a series of movements and these are represented as body parts changing in position over time. The licking behavior of a mouse, for example, in the formalin assay can take several distinct postural configurations, as the mouse may bend down towards the paw, hold the paw up or rapidly move the paw. In an example embodiment of the present disclosure, a machine learning (ML) model can identify and track multiple body parts on the subject. The use of a ML model may allow for calculation of many relative body-part-positions and use of a more complex representation of a subject's body for the formalin assay.

The genetic tractability of the mouse makes it an essential component in studies of pain and analgesia and therefore the development of automated nociception scoring in mice is important for large-scale studies. Recent innovations in machine learning allow for accurate classification of specific subject behaviors over the full-length of any recorded video. Advantages of such a system providing scalability, include the savings of time, labor and information with no restrictive sampling methods required. Refining the method of scoring the formalin assay results in greater reliability and reproducibility by improving the consistency of the measurements. Automated phenotyping also addresses the ethical requirements of replacement, reduction and refinement. The present disclosure, in some embodiments, includes supervised machine learning methods using recorded formalin assays performed on laboratory mice. The system was validated with extensive comparison to manual scoring. To assess the applicability to the widely used C57BL/6J derived strains and to the International Mouse Phenotyping Consortium's extensive collection of C57BL/6N derived deletion mutants, a comparison of both strains was performed.

The system of the present disclosure may provide many benefits. One of them may be providing refinement in examining nociceptive behaviors that improves reliability and reduces the need for replication of experiments. Another may be reduction in costs performing experiments and reduction in time in generating results of such experiments.

The system of the present disclosure, in some embodiments, includes three components / functionalities for automated phenotyping: (1) point detection (based on various body parts); (2) frame-level feature extraction; and (3) labeling of subject behavior. Configuration of the system in this manner enables the system to incorporate changes in tracking different body parts of the subject(s) and in determining different behaviors exhibited by the subject(s).

The system 100 of the present disclosure may operate using various components as illustrated in FIG. 1. The system 100 may include an image capture device 101, a device 102 and one or more systems 150 connected across one or more networks 199. The image capture device 101 may be part of, included in, or connected to another device (e.g., device 1600), and may be a camera, a high speed video camera, or other types of devices capable of capturing images and videos. The device 101, in addition to or instead of an image capture device, may include a motion detection sensor, infrared sensor, temperature sensor, atmospheric conditions detection sensor, and other sensors configured to detect various characteristics / environmental conditions. The device 102 may be a laptop, a desktop, a tablet, a smartphone, or other types of computing devices capable of displaying data, and may include one or more components described in connection with device 1600 below.

The image capture device 101 may capture video (or one or more images) of one or more subjects on whom the formalin assay is performed, and may send video data 104 representing the video to the system(s) 150 for processing as described herein. The system(s) 150 may include one or more components shown in FIG. 1, and may be configured to process the video data 104 to determine behaviors of the subject(s) over time. The system(s) 150 may determine output label data 130 associating one or more frames of the video data 104 with a label representing a behavior of the subject. The output label data 130 may be send to the device 102 for output to a user to observe the results of processing the video data 102.

Details of the components of the system(s) 150 are described below. The various components may be located on the same or different physical devices. Communication between the various components may occur directly or across a network(s) 199. Communication between the device 101, the system(s) 150 and the device 102 may occur directly or across a network(s) 199. One or more components shown as part of the system(s) 150 may be located at the device 102 or at a computing device (e.g., device 1600) connected to the image capture device 102.

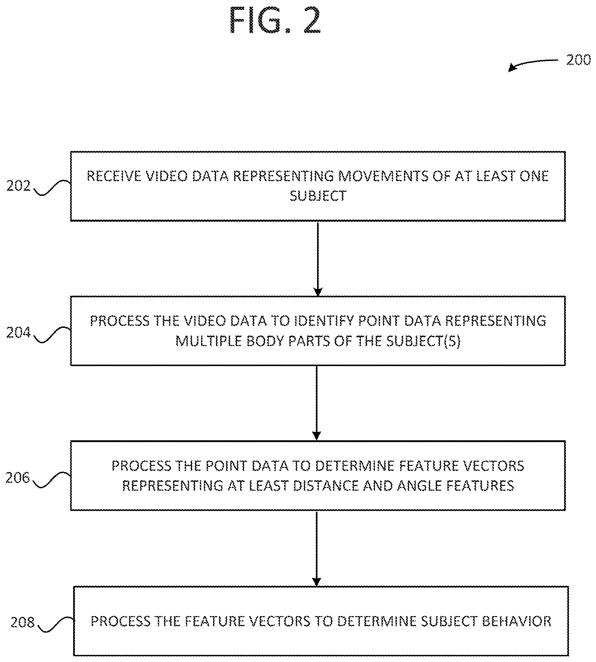

FIG. 2 is a flowchart illustrating a process 200 for analyzing video data 104 of a subject(s) to determine subject behavior, according to embodiments of the present disclosure. The steps of the process illustrated in FIG. 2 may be performed by the system(s) 150. In other embodiments, one or more steps of the process may be performed by the device 102 or a computing device associated with the image capture device 101.

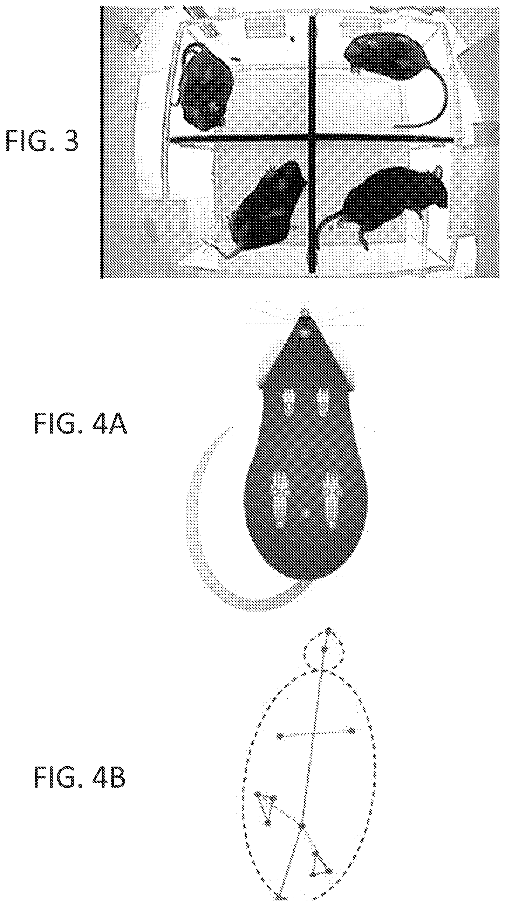

The system(s) 150 receives (202) video data 104 representing movements of at least one subject. In some cases, the subject is a mouse. In some embodiments, the video data 104 is a top-down camera view of a subject in an open field arena. In some cases, the video data 104 represents a video of multiple mice, for example, four mice, in (four) separate enclosures (as shown in FIG. 3). The video may capture the subject(s) movements over a period of time (e.g., one hour, 90 minutes, two hours, etc.). The subject(s) may have received formalin, and the captured movements may represent the subject's physical response (i.e. behavioral response) to the effects of the formalin. In some embodiments, the subject(s) may be receive other types of solutions / formulas to cause the subject to exhibit nociception behavior. Nociception is the neural processes of encoding and processing noxious stimuli. Nociception refers to a signal arriving at the central nervous system as a result of the stimulation of specialized sensory receptors in the peripheral nervous system called nociceptors. In some embodiments, a noxious stimuli used in a method of the invention is one or more of heat, high-intensity light, pressure, cold, physical injury, electricity, chemicals such as but not limited to: acetone and Complete Freund's Adjuvant (CFA), capsaicin, etc. These and other noxious stimuli may be used in certain embodiments of the invention to induce pain in a subject. In some embodiments, inducing the pain includes contacting the subject with one or more of: heat, light, pressure, cold, electricity, and a chemical agent. In certain embodiments of the invention, the subject is contacted with one or more of the heat, light, pressure, cold, electricity, and a chemical agent using a manner and amount sufficient to induce a desired pain and/or level of pain. In some embodiments, a means of inducing the pain in the subject includes inducing inflammation in the test subject. In some embodiments, a means of inducing the inflammation includes contacting the test subject with one or more of: heat, light, pressure, cold, electricity, and a chemical agent. In certain embodiments, the chemical agent includes one or more of formalin and acetone. In certain embodiments, a means of inducing the pain includes one or more of contacting the test subject with an effective amount of the pain-inducing agent, for example contacting the skill of the subject with an effective amount of a pain-inducing chemical agent, implanting a pain-inducing agent, such as but not limited to a stimulating electrode, a slow-release chemical agent, etc. into or onto the subject, injecting the subject with the chemical agent that induces pain, etc. Other art-known manners of pain-induction are suitable for use in methods of the invention, and amounts and levels of a pain-inducing agent can be selected using art-known means.

The video data 104 may correspond to a video captured by the device 101. In an example implementation, the video data 104 represents 30 frames per second with 704 x 480 pixels. In some cases, the video data 104 may correspond to images (image data) captured by the device 101 at certain time intervals, such that the images captures movements of the subject(s) over a period of time.

The system(s) 150, using a point tracker component 110, processes (204) the video data 104 to identify point data representing multiple body parts of the subject(s). The point tracker component 110 may be configured to identify various body parts of the subject(s). These body parts may be identified using point data, such that first point data may correspond to a first body part, second point data may correspond to a second body part, and so on. The point data may be, in some embodiments, one or more pixel locations / coordinates (x,y) corresponding to the body part. The point tracker component 110 may be configured to identify pixel locations corresponding to a particular body part within multiple frames of the video data 104. The point tracker component 110 may track movement of the particular body part during the video by identifying the corresponding pixel locations. The point data 112 may indicate the location of the particular body part during a particular frame of the video. The point data 112, provided to a feature extraction 115, may include the locations of all the body parts being identified and tracked by the point tracker component 110 over multiple frames of the video data 104.

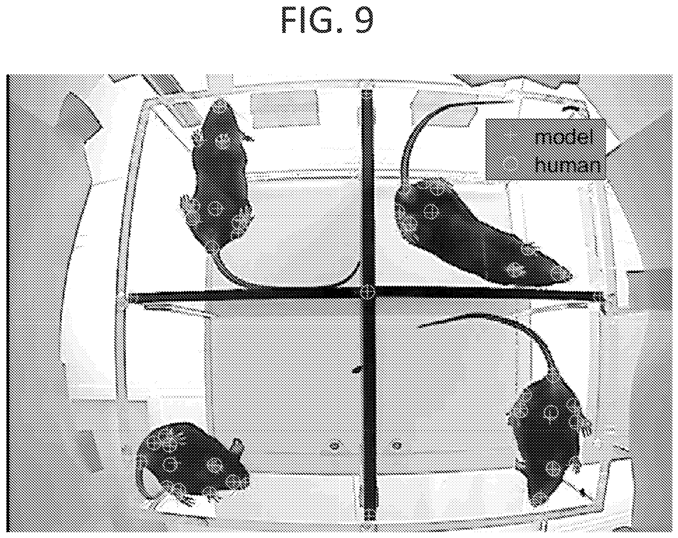

In some embodiments, where the subject is a mouse, the point tracker component 110 may identify and track the following body parts: mouth, nose, right front paw, left front paw, right hind paw, left hind paw, abdomen, and tailbase. FIGS. 4A and 4B illustrate example body parts being tracked using point data. FIGS. 4A shows the labels marked for a mouse subject (x, y pixel coordinates) for 12 points (mouth; nose; right front paw; left front paw; 3 points on each hind paw-outer, inner, and base; abdomen; and tailbase). FIG. 4B illustrates the 12 points per mouse that are the output of the key point tracker, shown here with "skeleton" connections and "body/head" circles for orientation. FIGS. 3 and 9 show an example of input video data. Each video captured 4 mouse subjects in 4 enclosures. The inner enclosure walls may be also marked, in some embodiments, for the point tracker component 110 to identify.

The point data 112 may be a vector, an array, or a matrix representing pixel coordinates of the various body parts over multiple video frames. For example, the point data 112 may be [frame1 = {mouth: (x1, y1); hind paw: (x2, y2)}], [frame2 = {mouth: (x3, y3); front paw: (x4, y4)}], etc. The point data 112, for each frame, may include in some embodiments at least 12 pixel coordinates representing 12 portions / body parts of the subject that the point tracker component 110 is configured to track.

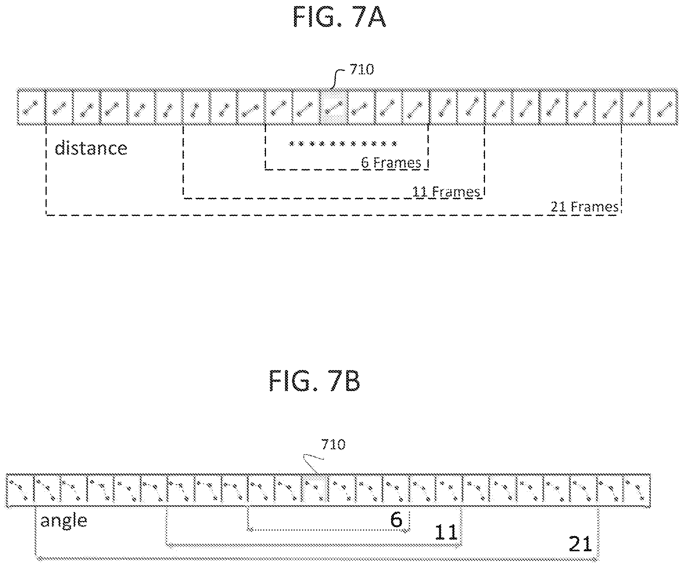

The system(s) 150, using the feature extraction component 115, processes (206) the point data 112 to determine feature vectors representing at least distance and angle features. The feature extraction component 115 may determine distances between various body parts of the subject(s) and generate one or more distance feature vectors 118. The feature extraction component 115 may determine first distance data between two (a first pair) of body parts, second distance data between another two (a second pair) of body parts, and so on, for multiple video frames. FIG. 7A illustrates an example distance feature vector including distance data representing a distance between two particular body parts over multiple video frames. The feature extraction component 115 may determine a first distance feature vector representing distances between a first pair of body parts over multiple video frames, a second distance feature vector representing distances between a second pair of body parts over multiple video frames, and so on.

The feature extraction component 115 may determine an angle between various body parts of the subject(s) and generate one or more angle feature vectors 116. The feature extraction component 115 may determine first angle data between three (a first trio) of body parts, second angle data between another three (a second trio) of body parts, and so on, for multiple video frames. FIG. 7B illustrates an example angle feature vector including angle data representing an angle between three particular body parts over multiple video frames. The feature extraction component 115 may determine a first angle feature vector representing angles between a first trio of body parts over multiple video frames, a second angle feature vector representing angles between a second trio of body parts over multiple video frames, and so on.

The system(s) 150, using a behavior classification component 120, processes (208) the feature vectors to determine subject behavior. In some embodiments, the behavior classification component 120 may process the distance feature vectors 118, the angle feature vectors 116, or both. The behavior classification component 120 may use one or more trained machine learning (ML) models to process the feature vectors. In some embodiments, the ML model may be a classifier configured to process the distance features and/or the angle features to determine whether the subject exhibits a particular behavior based on the location of one or more body parts relative to the other body parts. For example, in some cases, a mouse subject may exhibit nociception behavior by licking or biting a paw (e.g., a hind paw). The behavior classification component 120 may process the distance features and/or angle features to determine whether the mouse's paw is near or at the mouse's mouth indicating licking or biting behavior. The behavior classification component 120 may label each video frame with a particular behavior (e.g., licking, biting, no biting, no licking, etc.).

In some embodiments, the behavior classification component 120, using the ML model, may determine a probability (or a score, confidence score, etc.) corresponding to a likelihood of the subject exhibiting a particular behavior during the video frame. In this case, the output of the ML model may be a number between 0 and 1 or between 0 and 100, or any other such numerical ranges. The behavior classification component 120 may further process the output of the ML model to determine output labels 130 corresponding to the video frames of the video data 104.

In some embodiments, the behavior classification component 120 may be configured to perform binary classification, where a particular video frame may be classified into one of two behavior classes / categories (e.g., nociception behavior or no-nociception behavior; biting or no biting; licking or no licking; etc.). In this case, the behavior classification component 120 may associate a value such as, false or true, yes or no, 1 or 0, etc. with the video frame indicating whether the subject exhibits the particular behavior or not during that video frame.

In some embodiments, the behavior classification component 120 may be configured to perform multiclass or multinomial classification, where a particular video frame may be classified into one of three or more behavior classes / categories (e.g., no nociception, licking or biting). In some embodiments, the behavior classification component 120 may be configured to perform multi-label classification, where a particular video frame may be associated with more than one behavior class / category.

The behavior classification component 120 may include one or more machine learning (ML) models, including but not limited to, one or more classifiers, one or more neural networks, one or more probabilistic graphs, one or more decision trees, and others. In other embodiments, the behavior classification component 120 may include a rules-based engine, one or more statistical-based algorithms, one or more mapping functions or other types of functions / algorithms to determine whether particular distance and/or angle features indicate a particular behavior.

Although examples describe using the automated phenotyping methods and systems with mice subjects, it should be understood that the systems and methods herein can be configured to perform automated phenotyping for other types of subjects, such as, rats, rabbits, hamsters, etc. Additional information on subjects for which methods and systems of the invention can be used is provided elsewhere herein.

In some embodiments, the point tracker component 110 may implement one or more trained ML models configured to identify and track various body parts of a particular type of subject. The ML model(s) may be a neural network (e.g., deep neural network, convolutional neural network (CNN), recurrent neural network (RNN), etc.). In other embodiments, the ML model(s) of the point tracker component 110 may be other types of ML models. The ML model(s) of the point tracker component 110 may be configured for 3D markerless pose estimation based on transfer learning with deep neural networks.

In an example embodiment, the training data to configure the point tracker component 110 may be video frames from various videos of mice that have been administered formalin. The video frames may include movements of the mice over a period of time (e.g., 90 minutes). In some embodiments, the video frames may be from videos of mice that have not been administered formalin. Various body parts of the mice may be labeled in the training data. For example, the video frames may include 12 labeled points as illustrated in FIGS. 4A and 4B, corresponding to the following body parts: mouth, nose, right front paw, left front paw, 3 points on each hind paw, outer base, inner base, mid-abdomen, and tailbase. If the point tracker component 110 is being configured to track multiple mice within input video data, then the training data may be a video of multiple mice separated by different enclosures, and the training data may include labels identifying the enclosure walls. The training data may also include video frames corresponding to empty enclosures. Configuring the point tracker component 110 to track multiple mice within input video data eliminates the need for cropping or manipulating the input video data, and enables the system to perform phenotyping for multiple mice at a time.

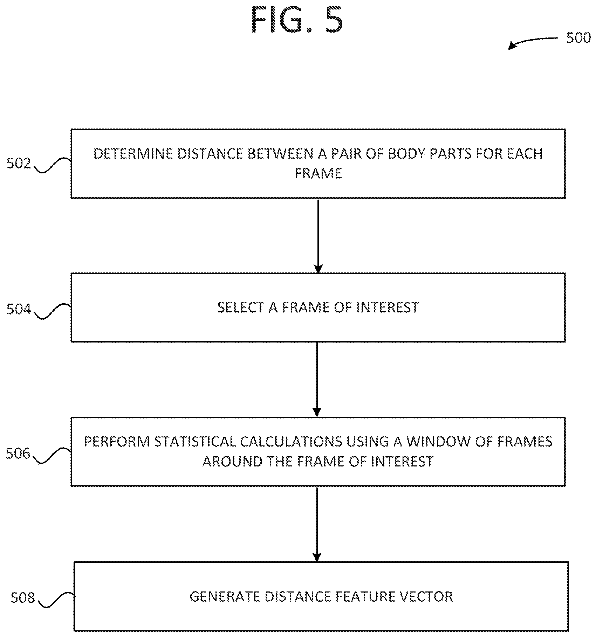

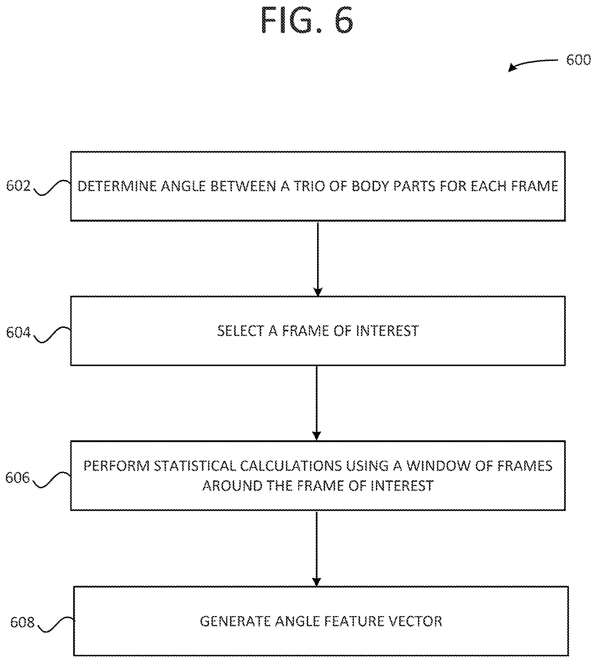

The point data 112 received by the feature extraction component 115 may include the (x, y) pixel coordinates for each specified body part location, as well as a likelihood estimate that is based on agreement of score-maps indicating the probability that the particular body-part is at the respective pixel. FIGS. 5 and 6 are flowcharts illustrating a process (500, 600) of determining feature vectors by the feature extraction component 115. The feature extraction component 115 determines (502) a distance between a pair of body parts of the subject for each video frame, where the body part is identified using the point data 112. For example, using 12 points on the subject body, the feature extraction component 115 may determine 66 body-part pairs. In some embodiments, the feature extraction component 115 may determine the Euclidean distance between the body parts of the pair of body parts. The feature extraction component 115 determines (602) an angle between a trio of body parts of the subject for each video frame, where the body part is identified using the point data 112. For example, using 12 points on the subject body, the feature extraction component 115 may determine 15 body-part trios. The feature extraction component 115 may determine an angle using a mid-point of the body-part trio.

The distances and angles determined at step 502 and 602 represent relative body-part location information for a single frame. The automated phenotyping system may be configured to detect change in the relative positions of the body-parts over time and identify that change as an action / movement by the subject. The automated phenotyping system may be configured to identify a behavior(s) based on a series of actions / movements by the subject. In this regard, the feature extraction component 115 may use consecutive video frames to observe the changes in distances and angles during a time period.

The feature extraction component 115 selects (504) a frame of interest, and performs (506) statistical calculations using distance data of a window of frames surrounding the frame of interest. Based on these statistical calculations, the feature extraction component 115 generates (508) a distance feature vector. The frame extraction component 115 selects (604) a frame of interest, and performs (606) statistical calculations using angle data of a window of frames surrounding the frame of interest. Based on these statistical calculations, the feature extraction component 115 generates (608) an angle feature vector.

In some embodiments, the feature extraction component 115 may use a window of 6 frames, selecting two video frames prior to the frame of interest and three video frames after the frame of interest. In other embodiments, the feature extraction component 115 may use a window of 11 frames, selecting five frames prior to the frame of interest and five frames after the frame of interest. In yet other embodiments, the feature extraction component 115 may use a window of 21 frames, selecting 10 frames prior to the frame of interest and 10 frames after the frame of interest. FIGS. 7A and 7B shows example feature vectors of one paired distance from points representing the body parts LeftFront (LF) to RightHindout (RHout) and one angle between points representing the body parts RightHindout (RHout), abdomen and LeftHindOut (LHout) over 24 consecutive frames with the frame of interest marked in the center. FIGS. 7A and 7B show the frame of interest 710 and the various windows of frames that the feature extraction component 115 may use. FIGS. 7A and 7B illustrate relative location measures (66 paired distances and 15 angles; only one of each shown in the figure) calculated for the body-parts for every video frame. The putative frame of interest is highlighted and the measures for the preceding and following frames are shown (24 consecutive frames). Statistical inputs may be calculated over windows of 3 different sizes (6, 11, and 21 frames) and these per-frame features may be used as the input to the behavior classification component 120.

The parameter value for each frame is calculated by moving the frame window and selecting the frame of interest accordingly. The parameter value for a frame of interest may be calculated by performing statistical analysis on the distance values and angle values of the frames within the window. In an example embodiment, the statistical analysis may include calculating the mean, standard deviation, median, and/or median absolute deviation using the distance values and angle values of the frames within the window. The determined parameter value is stored in the feature vector for the corresponding frame. In some embodiments, the feature extraction component 115 may use a GentleBoost classifier to determine the feature vectors. In some embodiments, the point data 112 indicating the likelihood estimations for the frame of interest may also be used to determine the feature vectors.

One distance feature vector 118 and one angle feature vector 116 may correspond to a first time period of the length of the video data 104, another distance feature vector 118 and another angle feature vector 116 may corresponding to a second time period of the length of the video data 104. Each value within the distance feature vector 118 and the angle feature vector 116 may correspond to a video frame (e.g., approximately 30 milliseconds) during the respective time period. A first value in the distance feature vector 118 and a first value in the angle feature vector 116 may correspond to the same first video frame.

In some embodiments, the feature extraction component 115 may determine feature data representing features corresponding to the video data 104 on a frame-level, and the feature data may be represented in a form other than a feature vector.

One or more ML models for the behavior classification component 120 may be configured using training data that may include labeled / annotated video data. The training video data may be labeled to indicate when a subject's movements (captured in the video) corresponds to a particular behavior. The training video data may include a first label associated with a video frame (e.g., 30 milliseconds duration of the video) and a second label associated with another video frame, where the first label indicates that the subject started exhibiting the particular behavior the automated phenotyping system is configured to detect and the second label indicates that the subject stopped exhibiting the particular behavior.

The configuration of the training video data may depend on the configuration of the automated phenotyping system. In the case that the behavior classification component 120 is configured to classify the subject's behavior based on whether the subject is exhibiting the behavior or not, the training video data may include labels associated with video frames where the subject is exhibiting the behavior. For example, when the automated phenotyping system is configured to detect licking behavior in a mouse, the training video data may include labels associated with the video frames during which the mouse is licking its hind paw. In the case that the behavior classification component 120 is configured to classify the subject's behavior based on the type of behavior the subject is exhibiting, the training video data may include a first type of label associated with video frames during which the subject is exhibiting the first type of behavior, a second type of label associated with the video frames during which the subject is exhibiting the second type of behavior, and so on.

In some embodiments, the behavior classification component 120 may be configured to detect specific movements by the subject to indicate a particular behavior. For example, the behavior classification component 120 may be configured to detect licking of a right hind paw by a mouse subject to indicate licking behavior (where the right hind paw is injected with formalin). In other embodiments, the behavior classification component 120 may be configured to detect different types of movements by the subject to indicate the particular behavior. For example, the behavior classification component 120 may be configured to detect licking of either of the right or left hind paw by a mouse subject to indicate licking behavior. In some embodiments, any contact between a hind paw and a mouth (e.g., touching of the hind paw by the mouth, proximity of the hind paw to the mouth, licking of the hind paw by the mouth, biting of the hind paw by the mouth, etc.) may be labeled as nociception behavior.

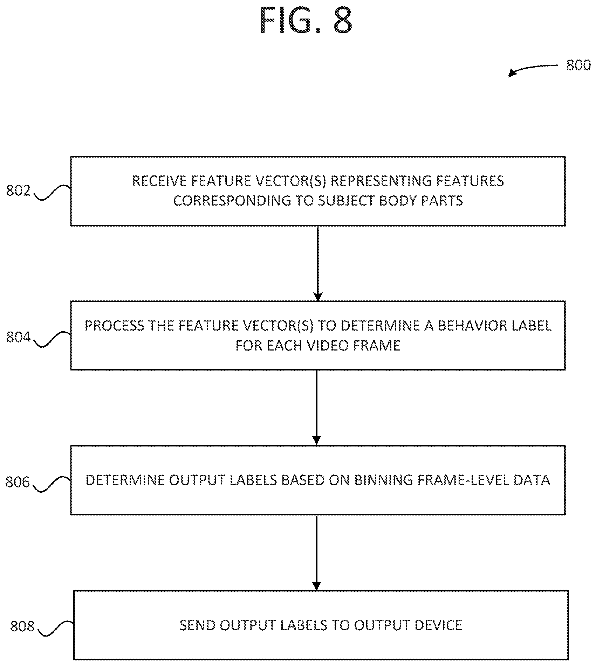

FIG. 8 is a flowchart of a process 800 that may be performed by the behavior classification component 120. The behavior classification component 120 receives (802) feature vectors (e.g., 116, 118) or feature data determined by the feature extraction component 115. The behavior classification component 120 processes (804) the feature vectors to determine a label for each video frame in the video data 104. Based on the system configuration, the label may indicate the type of behavior exhibited by the subject during the video frame. Because the feature vectors includes values determined by performing statistical analysis using features of the frames surrounding the frame of interest, the behavior classification component 120 is provided context with respect to subject's movements prior to and after the particular frame of interest. The behavior classification component 120 may process the feature vectors using one or more ML models, and the output of the ML model(s) may be a score / probability indicating a likelihood of each video frame corresponding to a particular behavior or may be a probability distribution / vectors of scores for each video frame. For example, in the case the behavior classification component 120 is configured to determine whether the subject is exhibiting a behavior, a first video frame may be associated with a first score, a second video frame may be associated with a second score, etc. In another example, in the case the behavior classification component 120 is configured to determine whether the subject is exhibiting one of two behaviors, a first video frame may be associated with a score vector {score1, score2}, where the score1 may indicate the likelihood of the subject exhibiting a first behavior and score2 may indicate the likelihood of subject exhibiting a second behavior. In some embodiments, the ML model(s) outputs a label (e.g., true or false, yes or no, 0 or 1, etc.) for each video frame indicating whether or not the subject is exhibiting a behavior.

The behavior classification component 120 determines (806) output labels based on binning of the outputs of the ML model(s), and sends (808) the output labels to the device 102 for display to a user. The behavior classification component 120 may be configured to bin the output of the ML model(s) based on a fixed number of video frames, a length of time, or the ML model(s) output. For example, the behavior classification component 120 may bin the ML model output into 5-minute bins, where the label associated with the 5-minute bin is determined using the scores / labels corresponding to the video frames included in the 5-minute bin. The bin size may depend on the type of information required by a user, the type of subjects, the type of behaviors to be detected, and other factors.

The automated phenotyping system may be configured to process a single video capturing movements of multiple subjects. In this case, the point tracker component 110 is configured to identify the multiple subjects, identify and track body parts of each of the subjects, and generate point data 112 corresponding to each of the subjects. The point tracker component 110 may generate first point data 112 corresponding to a first subject, second point data 112 corresponding to a second subject, and so on. The feature extraction component 115 is configured to process the point data 112 and generate feature vectors corresponding to each of the subjects. The feature extraction component 115 may generate a first feature vector corresponding to a first subject, a second feature vector corresponding to a second subject, and so on. The behavior classification component 120 is configured to process feature vectors corresponding to multiple subjects and generate output labels indicating when / whether the subjects exhibit a particular behavior.

The automated phenotyping system described herein may be an efficient manner of scoring / classifying subject behavior for various assays. The classification performed by the system described herein may be comparable, in some respects, to manual classification of subject behavior. Additionally, the automated phenotyping system can be used to process behavior for multiple subjects at a time, enabling scalability and processing of different genetic strains.

Although the automated phenotyping system is described as including a point tracker component, a feature extraction component and a behavior classification component, it should be understood that fewer or more components or different types of components (of different types of ML models or techniques) may be used to perform the functionalities described herein. For example, a different way for tracking body parts in the video data can be used, a different window size for processing distance and angle features may be used, a different type of ML model may be used for processing the feature vectors, etc.

In some aspects, the localization of individual body-parts may result in a reduction of data to be processed (e.g., from 337,920 pixels per video frame to 159 numbers of interest (x, y coordinates and likelihoods of 53 points)), making the task of tracking body-parts over lengthy videos manageable. The position of body-parts relative to each other, measured in distances and angles as described herein, may be an efficient way to generate body-part information for each video frame. The average errors of localization of body-points of interest may be below five pixels. Because the behavior classification is conducted over many thousands of frames (e.g., 153,000 frames for 85 minutes), even if a few frames contain larger errors (with respect to body parts identification) it may have negligible impact on the overall behavior classification. The system described herein uses temporal windows to assess change across time to reduce the impact of a single frame and to help smooth disparities between consecutive frames.

In some embodiments, the behavior classification component 120 may be configured to detect a subject behavior involving licking or biting of a body part (e.g., a paw). In such embodiments, the behavior classification component 120 may employ one or more ML models trained to detect licking or biting behavior. In other embodiments, the behavior classification component 120 may be configured to detect a subject behavior involving shaking of a body part (e.g., a paw). In such embodiments, the behavior classification component 120 may employ one or more ML models trained to detect shaking behavior. In yet other embodiments, the behavior classification component 120 may be configured to detect a subject behavior involving rearing supported by a wall of an arena within which the subject is located. In such embodiments, the behavior classification component 120 may employ one or more ML models trained to detect rearing behavior.

In yet other embodiments, the behavior classification component 120 may be configured to detect different types of behaviors: licking or biting behavior, shaking behavior, and rearing behavior. In such embodiments, the behavior classification component 120 may employ one or more ML models trained to detect licking or biting behavior, one or more separate ML models trained to detect shaking behavior, and one or more separate ML models trained to detect rearing behavior. In other such embodiments, the behavior classification component 120 may employ one or more ML models that is trained to detect all the different behaviors: licking or biting behavior, shaking behavior, and rearing behavior.

In some embodiments, the system(s) 150 may be configured to determine metrics relating to the subject's behavior. For example, the system(s) 150 may determine a number of times the subject exhibits licking or biting behavior during the video, an amount of time the subject exhibits licking or biting behavior during the video, and a length of time each licking or biting behavior bout. As a further example, the system(s) 150 may determine a number of times the subject exhibits shaking behavior during the video, an amount of time the subject exhibits shaking behavior during the video, and a length of time each shaking behavior bout. As yet a further example, the system(s) 150 may determine a number of times the subject exhibits rearing behavior during the video, an amount of time the subject exhibits rearing behavior during the video, and a length of time each rearing behavior bout.

**In** some embodiments, the system(s) 150 may be configured to determine various per-video frame features corresponding to the subject. Such per-video frame features may include, but are not limited to, open field metrics, gait metrics, and other heuristic metrics. One or more of the foregoing features may be used by the behavior classification component 120 to detect a subject behavior. **In** other embodiments, the foregoing features may not be considered by the behavior classification component 120, and may instead be used to compare a subject (e.g., having a first genotype or phenotype) to another subject (e.g., having a second different genotype or phenotype).

The open field metrics may correspond to the subject movements within an open field arena, and may include, but are not limited to, time spent by the subject at / near a center of the arena, time spent by the subject at / near a periphery of the arena, time spent by the subject at / near a corner of the arena, a distance between the subject's location and the center of the arena, a distance between the subject's location and the periphery of the arena, a distance between the subject's location and the corner of the arena, a number of times the subject is grooming, and an amount of time the subject is grooming. Such open field metrics may be determined using the point data 112.

The gait metrics may correspond to the subject's gait / walk, and may include, but are not limited to, step width, step length, stride length, speed, angular velocity, limb duty factor (e.g., based on a stance time / an amount of time a paw is in contact with the ground during a stride interval, and the total time of the stride interval), lateral displacement of nose (in view of a center spine vector of the subject), lateral displacement of tail base (in view of a center spine vector of the subject), lateral displacement of tail tip (in view of a center spine vector of the subject), and temporal symmetry (e.g., similarities in stride features during a set of video frames). Such open field metrics may be determined using the point data 112.

The other heuristics features may correspond to other movements of the subject, and may include, but are not limited to, an amount of time the subject is frozen / does not move, a number of times the subject is frozen, a number times the subject has continuous movement / gait, and an amount of time the subject has continuous movement / gait. Such heuristics features may be determined using the point data 112, the open field metrics and/or the gait metrics.

### Stitching Video Feeds

In some embodiments, the video data 104 may be generated using multiple video feeds capturing movements of the subject from multiple different angles / views. The video data 104 may be generated by stitching / combining a first video of a top view of the subject and a second video of a side view of the subject. The first video may be captured using a first image capture device (e.g., device 101a) and the second video may be captured using a second image capture device (e.g., device 101b). Other views of the subject may include a right side view, a left side view, a top-down view, a bottom-up view, a front side view, a back side view, and other views. Videos from these different views may be combined to generate the video data 104 to provide a comprehensive / expansive view of the subject's movements that may result in more accurate and/or efficient classification of subject behavior by the automated phenotyping system. In some embodiments, videos from different views may be combined to provide a wide field of view with a short focal distance, while preserving a top-down perspective over the entirety of the view. In some embodiments, the multiple videos from different views may be processed using one or more ML models (e.g., neural networks) to generate the video data 104. In some embodiments, the system may generate 3D video data using 2D video / images.

In some embodiments, the videos captured by the multiple image capture devices 101 may be synced using various techniques. For example, the multiple image capture devices 101 may be synced to a central clock system and controlled by a master node. Synchronization of multiple video feeds may involve the use various hardware and software such as an adapter, a multiplexer, USB connections between the image capture devices, wireless or wired connections to the network(s) 199, software to control the devices (e.g., MotionEyeOS), etc.

In an example embodiment, the image capture device 101 may be an ultra-wide-angle lens (i.e., a FishEye lens) that produces strong visual distortion intended to create a wide panoramic or hemispherical image, and capable of achieving extremely wide angles of view. In an example implementation, the system to capture the videos for video data 104 may include 4 FishEye lens cameras connected to 4 single-board computing devices (e.g., a Raspberry Pi), and an additional image capture device to capture a top-down view. The system may synchronize these components using various techniques. One technique involves pixel / spatial interpolation, for example, where a point-of-interest (e.g., a body part on the subject) is located at (x, y), the system identifies, with respect to time, a position within the top-down view video along the x and y axes. In an example, the pixel interpolation for the x-axis may be calculated by the single-board computing device per the following equation: $\left(Pi offset Δ X/Pi offset Δ T\right) * \left(top-down view offset Δ T\right) + the initial point \left(x\right)$ The equation then may be used to calculate the point-of-interest position for the y axis. **In** some embodiments, to address lens distortion during video calibration, padding may be added to one or more video feeds (instead of scaling the video feed).

### Subjects

Some aspects of the invention include use of automated phenotyping methods with a subject. As used herein, a the term "subject" may refer to a human, non-human primate, cow, horse, pig, sheep, goat, dog, cat, pig, bird, rodent, or other suitable vertebrate or invertebrate organism. In certain embodiments of the invention, a subject is a mammal and in certain embodiments of the invention a subject is a human. In some embodiments a method of the invention may be used in a rodent, including but not limited to a: mouse, rat, gerbil, hamster, etc. In some embodiments of the invention, a subject is a normal, healthy subject and in some embodiments, a subject is known to have, at risk of having, or suspected of having a disease or condition. The terms "subject" and "test subject" may be used interchangeably herein.

As a non-limiting example, a subject assessed with an automated phenotyping method of the invention may be a subject that is an animal model for a pain disease or condition such as a model for one or more of: inflammatory pain, neuropathic pain, muscle pain, joint pain, chronic pain, visceral pain, cancer pain, and postoperative pain. Additional models of chronic pain are suitable for use in methods and systems of the invention are known in the art, see for example: Barrot M. Neuroscience 2012; 211: 39-50; Graham, D.M., Lab Anim (NY) 2016; 45: 99-101; Sewell, R.D.E., Ann Transl Med 2018; 6: S42. 2019/01/08; and Jourdan, D., et al., Pharmacol Res 2001; 43: 103-110, the contents of which are incorporated herein by reference in their entirety.

In some embodiments a subject may be monitored using an automated phenotyping method or system of the invention, wherein pain is not induced in the subject. For example, a test subject that is a model for a pain condition may not be administered an external pain-inducing action such as an injection of a chemical agent or exposure to a pain-inducing heat, light, pressure, etc. In some embodiments of the invention a test subject is an animal model for neuropathic pain and the test subject is monitored using an automated phenotyping method and/or system of the invention without inducing additional pain in the test subject. In certain embodiments of the invention, a test subject that is an animal model of a pain condition may be used to assess the test subject's response to the pain condition. In addition, a test subject that is an animal model of a pain condition may be administered a candidate therapeutic agent or method, monitored using an automated phenotyping method or system of the invention and the efficacy of the candidate therapeutic agent or method to reduce the pain of the pain condition in the test subject can be determined.

In some embodiments of an automated phenotyping method of the invention, a subject is a wild-type subject. As used herein the term "wild-type" means to the phenotype and/or genotype of the typical form of a species as it occurs in nature. In certain embodiments of the invention a subject is a non-wild-type subject, for example, a subject with one or more genetic modifications compared to the wild-type genotype and/or phenotype of the subject's species. In some instances a genotypic/phenotypic difference of a subject compared to wild-type results from a hereditary (germline) mutation or an acquired (somatic) mutation. Factors that may result in a subject exhibiting one or more somatic mutations include but are not limited to: environmental factors, toxins, ultraviolet radiation, a spontaneous error arising in cell division, a teratogenic event such as but not limited to radiation, maternal infection, chemicals, etc.

In certain embodiments of methods of the invention, a subject is a genetically modified organism, also referred to as an engineered subject. An engineered subject may include a pre-selected and/or intentional genetic modification and as such exhibits one or more genotypic and/or phenotypic traits that differ from the traits in a non-engineered subject. In some embodiments of the invention routine genetic engineering techniques can be used to produce an engineered subject that exhibits genotypic and/or phenotypic differences compared to a non-engineered subject of the species. As a non-limiting example, a genetically engineered mouse in which a functional gene product is missing or is present in the mouse at a reduced level and a method or system of the invention can be used to assess the genetically engineered mouse phenotype, and the results may be compared to results obtained from a control (control results).

### Controls and candidate compound testing and screening

Results obtained for a subject using an automated phenotyping method or system of the invention can be compared to control results. Methods of the invention can also be used to assess a difference in a phenotype in a subject versus a control. Thus, some aspects of the invention provide methods of determining the presence or absence of a change in an activity in a subject compared to a control. Some embodiments of the invention include using automated phenotypic methods of the invention to identify phenotypic characteristics of a disease or condition and in certain embodiments of the invention automated phenotyping is used to assess an effect of a candidate therapeutic compound on a subject.

Results obtained using an automated phenotyping method or system of the invention can be advantageously compared to a control. In some embodiments of the invention one or more subjects can be assessed using an automated phenotyping method followed by retesting the subjects following administration of a candidate therapeutic compound to the subject(s). The terms "subject" and "test subject" may be used herein in relation to a subject that is assessed using a method or system of the invention, and the terms "subject" and "test subject" are used interchangeably herein. In certain embodiments of the invention, a result obtained using an automated phenotyping method to assess a test subject is compared to results obtained from the automated phenotyping methods performed on other test subjects. In some embodiments of the invention a test subject's results are compared to results of the automated phenotyping performed on the test subject at a different time. In some embodiments of the invention, a result obtained using an automated phenotyping method to assess a test subject is compared to a control result.

As used herein a control result may be a predetermined value, which can take a variety of forms. It can be a single cut-off value, such as a median or mean. It can be established based upon comparative groups, such as subjects that have been assessed using an automated phenotyping system or method of the invention under similar conditions as the test subject, wherein the test subject is administered a candidate therapeutic agent and the comparative group has not been contacted with the candidate therapeutic agent. Another example of comparative groups may include subjects known to have a disease or condition and groups without the disease or condition. Another comparative group may be subjects with a family history of a disease or condition and subjects from a group without such a family history. A predetermined value can be arranged, for example, where a tested population is divided equally (or unequally) into groups based on results of testing. Those skilled in the art are able to select appropriate control groups and values for use in comparative methods of the invention.

A subject assessed using an automated phenotyping method or system of the invention may be monitored for the presence or absence of a change that occurs in a test condition versus a control condition. As non-limiting examples, in a subject, a change that occurs may include, but is not limited to one of more of: a frequency of movement, a licking behavior, a response to an external stimulus, etc. Methods and systems of the invention can be used with test subjects to assess the effects of a disease or condition of the test subject and can also be used to assess efficacy of candidate therapeutic agents. As a non-limiting example of use of method of the invention to assess the presence or absence of a change in a test subject as a means to identify efficacy of a candidate therapeutic agent, a test subject known to have a pain condition is assessed using an automated phenotyping method of the invention. The test subject is then administered a candidate therapeutic agent and assessed again using the automated phenotypic method. The presence or absence of a change in the test subject's results indicates a presence or absence, respectively, of an effect of the candidate therapeutic agent on the pain condition.

It will be understood that in some embodiments of the invention, a test subject may serve as its own control, for example by being assessed two or more times using an automated phenotyping method of the invention and comparing the results obtained at two or more of the different assessments. Methods and systems of the invention can be used to assess progression or regression of a disease or condition in a subject, by identifying and comparing changes in phenotypic characteristics in a subject over time using two or more assessments of the subject using an embodiment of a method or system of the invention.

### Examples

### Example 1. Model development: data training, testing, and model validation

### Methods

### Animal care

Mice were single sex, group-housed (3-5) with *ad lib* water and food under a 12-hour light-dark schedule. Experiments were conducted in the light phase. Video data from 166 mice were used in training, testing, and validation of the model (Jackson Laboratory: C57BL/6NJ = JR005304: male n = 53, female = 37: C57BL/6J = JR000664: male = 46, female = 30). Mice (age 11-17 weeks) were tested in 25 sessions; at the conclusion of each experimental session all mice were euthanized by cervical dislocation. All procedures and protocols were approved by The Jackson Laboratory Animal Care and Use Committee, and were conducted in compliance with the National Institutes of Health Guideline for Care and Use of Laboratory Animals.

### Video data collection

Video data of mouse behavior in response to a hind paw formalin injection were collected and used in training, testing and validation of the automated phenotyping system. A clear acrylic enclosure (22 cm L x 21.6 cm W x 12.7 cm H; IITC Life Science, Woodland Hills, CA) containing four testing arenas separated by opaque black walls (as shown in FIGS. 4 and 9) was placed on a clear glass surface. A black-and-white Dinion video camera (Bosch, Farmington Hills, MI) was placed directly below (16 cm) the glass floor of the enclosure to provide the best view of the paws and recording, under the control of Noldus media recorder v4 software (Noldus, Leesburg, VA), began with the empty enclosure. Four enclosures, each with one dedicated camera, were set up such that a total of 16 mice could be run simultaneously. The lighting varied between the four enclosures but was optimized to reduce glare and reflections with the addition of a white polycarbonate cover for the top of each enclosure (23.5 cm L x 12.1 cm W x 1 cm H; manufactured in-house). Video was recorded (30 frames per second: 704 X 480 pixels) for 90 minutes after the last mouse entered the arena. Video was extended beyond 60 minutes to ensure that any strain differences in the timing of peak behavior would be captured.

Formalin was administered while the mice were under anesthesia to maximize the consistency of both the injection site and the volume delivered and to reduce stress for the mice. The right hind paw of the mouse was injected (intra-plantar) with 30µl of 2.5% Formalin solution in saline [formaldehyde solution (Sigma-Aldrich, St. Louis, MO; sterile saline solution (Henry Schein, Dublin, OH; under gas anesthesia (4% isoflurane; Henry Schein Isothesia, Dublin, OH). The mouse was then transferred into the first testing arena and the procedure was repeated with the next three mice for this enclosure. Typically, mice regained consciousness from the anesthesia within one minute of being placed in the testing arena, and were fully ambulatory within three minutes.

### (1) Training data for point detection

To create a training set for point detection (the point tracker component 110), frames were pseudo-randomly selected from eight videos of mice covering the four different enclosures and ensuring representation of early (up to 30 minutes), middle (30-60) and late (60-90) portions of the recordings. Labels were manually applied to the desired points on 370 frames. Each mouse was labeled with 12 points as shown in FIGS. 4A and 4B (mouth, nose, right front paw, left front paw, 3 points on each hind paw -outer, inner and base, mid-abdomen, and tailbase), and the inner walls of each arena were labeled with 5 points (as shown in FIG. 4A). Each frame was thus labeled with a total of 53 points. The point tracker was trained to find all 53 points per frame and therefore it was not necessary to crop or manipulate the video frame to locate a single arena. The location of the grid walls were included for training purposes to verify that all 12 mouse points were located within a single arena. Any point missing or obscured was labelled as location x = 0, y = 0 and all labelled frames were visually re-checked for accuracy. Examples of empty arenas were included in training. To increase the number of frames for training, the 370 frames were reflected and rotated so that every mouse appeared in each of the four locations for a total of 1480 labelled frames. To increase the variability in lighting conditions used for training, approximately 11% of the 1480 frames were augmented with the addition of Gaussian noise (40 frames) or alterations of contrast (39 frames), brightness (39 frames) or gamma filtering (40 frames) (Table 1, below). The augmented frames were pseudo-randomly chosen and distributed evenly across the original 370 and each of the reflection and rotation conditions. After these adjustments the set of labelled frames was divided randomly into a training set (85%) and a test set for validation (15%).

**Table 1. Adjustments made to approximately 11% of images.**

| **Gaussian Noise** | | **Brightness** | | **Contrast** | | **Gamma Filter** | |
|---|---|---|---|---|---|---|---|
| Parameter value | Number of frames | Parameter value | Number of frames | Parameter value | Number of frames | Parameter value | Number of frames |
| 10% add | 20 | 220 | 8 | 220 | 8 | 0.3 | 12 |
| 15% add | 20 | 235 | 12 | 235 | 12 | 0.5 | 12 |
| | | 285 | 12 | 285 | 12 | 1.5 | 12 |
| | | 300 | 7 | 301 | 7 | 2 | 4 |

### (1)(a) Pose estimation

The point tracker component 110 may take advantage of a pre-trained Residual Network (ResNet50) for body-part detection. Residual network architecture uses convolution layers to learn specific visual features and the skipping function minimizes information loss, thereby enhancing extraction of global rules.

Tensorflow^{™} was used to train the ResNet50 architecture on a Tesla P100 GPU (Nvidia, Santa Clara, CA). The model of the point tracker component 110 was trained for 750,000 iterations attaining training accuracy of 1.9 pixels and test error of approximately 4.4 pixels error averaged over all test frames and points. FIG. 9 shows an example of a single test frame (average error of 2.4 pixels): in arena 4, the right front paw is missed by 4.3 pixels which is the approximate size of the average error over all test frames. The stability of performance was verified by repeating the training with a different training and testing set (train error 1.9 and test error 4.3).

The trained model was locked and subsequently used to track the experiment videos. The videos were approximately 100-120 minutes long (ranging from 1.6 to 2.2 GB); each frame was 337,920 pixels (704 X 480), and the speed to label 53 points varied between 36-37 frames per second (on a Tesla GPU). Tracking of the four mice in a video was effectively slightly faster than the video recording speed of 30 frames a second.

### (2) Frame feature extraction

Using the feature extraction component 115 and the (x, y) pixel coordinates for each specified body part, as well as a likelihood estimate that is based on agreement of score-maps indicating the probability that this body-part is at this pixel, feature vectors are generated. When the arena was empty, all 12 points were located with very low probabilities (e.g., > 0.0001) and as soon as the mouse was placed in the arena, all points increased in likelihood estimations. The threshold of an average probability of 0.8 across the 12 points was used to indicate that a mouse was present.

Because the number of mice per enclosure varied (1 to 4), each mouse was classified independently. The 12 key points of interest (shown in FIGS. 4A and 4B) were used to generate pairwise Euclidean distances between body-parts (66 pairs) and the angles between selected trios of body-parts (15 angles, shown in Table 2 below). FIGS. 7A and 7B show example feature vectors of one paired distance from points representing the body parts LeftFront (LF) to RightHindout (RHout) and one angle between points representing the body parts RightHindout (RHout), abdomen and LeftHindOut (LHout) over 24 consecutive frames with the frame of interest marked in the center.

**Table 2. Angles calculated between 3 body-part points with the angle subtended around the midpoint.**

| **Point1** | **Midpoint** | **Point2** |
|---|---|---|
| nose | RHout | LHout |
| nose | RightHindBase (RHbase) | LeftHindBase(LHbase) |
| nose | RightHindIn (RHin) | LeftHindIn (LHin) |
| mouth | RHout | LHout |
| mouth | RHbase | LHbase |
| mouth | RHin | LHin |
| tailbase | RHout | LHout |
| tailbase | RHbase | LHbase |
| tailbase | RHin | LHin |
| abdomen | RHout | LHout |
| abdomen | RHbase | LHbase |
| abdomen | RHin | LHin |
| LF | RHout | RightFront (RF) |
| LF | RHbase | RF |
| LF | RHin | RF |

To test the automated phenotyping system, frame windows sizes of 6, 11, and 21 frames (200, 367, 700 ms) were chosen for licking behavior. Each window was moved along the vector and the statistics of the parameter were calculated within that time frame. A total of 1047 different metrics were calculated for each video frame and served as the input data to the behavior classification component 120. Statistical metrics were mean, standard deviation, median and median absolute deviation for each distance pair. A second measure of distance was included that reported no value (NaN) if the mouth or nose fell below 0.1 likelihood and the mean was calculated for this and for the angles. The 12 likelihood estimations for the frame of interest were also included as input without windowing.

### (3) Behavior classification

For the behavior classification component 120, training data were taken from 50 different videos, to cover all enclosures, arenas and sizes or sex of mice. A total of 9300 frames were used for training with no more than 10 seconds (approximately 300 frames) per video. The video data was annotated on a frame-by-frame basis to indicate onset and offset video frames of licking behavior. No distinction was made between licking and biting behaviors, in this case, and any contact between the mouth and the right hind paw was labeled as licking. To obtain a well-balanced training set, frames were selected using stratified random sampling from clear licking (22%) and non-licking video segments (78%). The bias towards no licking behavior was intentional because this behavior does not occur equally in an input runtime video.

### (3)(a) GentleBoost Classifier Models

GentleBoost (gentle adaptive boost) is an ensemble supervised learner based on minimizing exponential loss using decision trees. The GentleBoost algorithm is well suited for a dichotomous categorical response. The classifier used 30 weighted learners, each of which fitted a regression model to the predictors and labels using a maximum of 20 splits and a learning rate of 0.1. Five-fold cross validation was used to limit overleaming and to provide estimates of training. Because the large number of inputs had significant redundancy, the GentleBoost model was also trained with the implementation of Principal Component Analysis (PCA), accounting for either 99% (65 inputs) or 95% (11 inputs) of variation.

### Results

### Testing GentleBoost Classifier Models

Table 3 shows the results of all the tested classifiers for precision (proportion of frames correctly classified as licking), recall (proportion of licking frames correctly identified), false positives (proportion of incorrectly labeled frames as licking) and total overall accuracy. High values in precision-recall dimensions indicate that the model is able to correctly identify licking without missing occurrences of behavior, regardless of how rarely the behavior occurs, which is particularly useful when the two behaviors are unequally distributed. Low false positive rates indicated that when licking is not present then the model does not report the behavior; this shows that the model does not label everything as licking in order to prevent missing the behavior. The GentleBoost model performed well on all metrics. PCA reduction of parameters resulted in diminished performance for precision and recall, with a slight increase in false positive rates (see Table 3).

**Table 3. Results of classifier models on the validation dataset. Precision = true positive / (true positive + false positive; or what proportion of frames identified as licking are truly licking?). Recall = true positive / (true positive + false negative; or what proportion of true licking frames were found?). False positive = what proportion of "no licking" frames incorrectly identified as licking.**

| **Classifier** | **Precision (%)** | **Recall (%)** | **False Positive (%)** | **Total Accuracy (%)** |
|---|---|---|---|---|
| 12 point GentleBoost | 96 | 95 | 1 | 97.9 |
| 12 point GentleBoost PCA 99% | 94 | 90 | 2 | 96.7 |
| 12 point GentleBoost PCA 95% | 93 | 89 | 2 | 96.2 |
| 8 point GentleBoost | 95 | 94 | 1 | 97.6 |
| 8 point GentleBoost PCA 99% | 92 | 89 | 2 | 96.4 |
| 8 point GentleBoost PCA 95% | 94 | 87 | 2 | 95.6 |
| 5 point GentleBoost | 93 | 93 | 2 | 96.9 |
| 5 point GentleBoost PCA 99% | 92 | 88 | 2 | 95.7 |
| 5 point GentleBoost PCA 95% | 87 | 85 | 3 | 94.1 |
| 12 point Fine KNN | 95 | 94 | 1 | 97.6 |
| 12 point Ensemble KNN | 95 | 94 | 1 | 97.6 |
| 12 point Cubic SVM | 93 | 92 | 2 | 96.8 |

To determine if 12 points for the body parts were necessary for optimal performance the GentleBoost model was retrained with inputs calculated from 8 points (removed both front paws and the inner point on both hind paws) or 5 points (also removed mouth and the outer point from both hind paws). Reducing the number of points resulted in a slight loss of performance but the 8 point model was very similar to the full 12 point model. PCA for the 8-point and 5-point models resulted in clear loss of precision and recall and a small increase in false positive rates.

The full GentleBoost classifier using all 12 mouse body-parts and all statistical parameters (1047 inputs), had the best performance (see Table 3). However, labeling all 12 points for training the tracking module is a time-consuming endeavor and could be reduced to fewer points if the slight loss of performance was an acceptable trade-off. The loss of performance with PCA is not worth the efficiency benefits as the cost of evaluating the full 12 point classifier is low (prediction speed approximately 10,000 observations per second). Using Matlab on a laptop to open excel file data, calculate inputs, classify behavior, calculate bins and save results to 3 different formats (HDF5 file, excel spreadsheet and backup Matlab output structure file) took approximately 20-25 seconds per mouse. A smaller parameter list is more efficient but even a small loss in accuracy in detecting licking does not seem warranted given the low cost of keeping all parameters.

### Other Classifier Models

Two other classifiers were tested using all the 1047 input parameters (see Table 3): a k-Nearest Neighbor (kNN) classifier (neighbors = 1, Euclidian distance, equal distance weight, ties broken to smallest; prediction speed 110 observations/second) and an ensemble subspace kNN classifier (30 learners, subspace dimension = 624, prediction speed 8.7 observations/second). Both performed almost as well as the full GentleBoost model but were less efficient in implementation of prediction. A Support Vector Machine (SVM) with a cubic kernel was more efficient than the kNN models (1600 observations/second) but slightly less accurate.

### Model Parameters

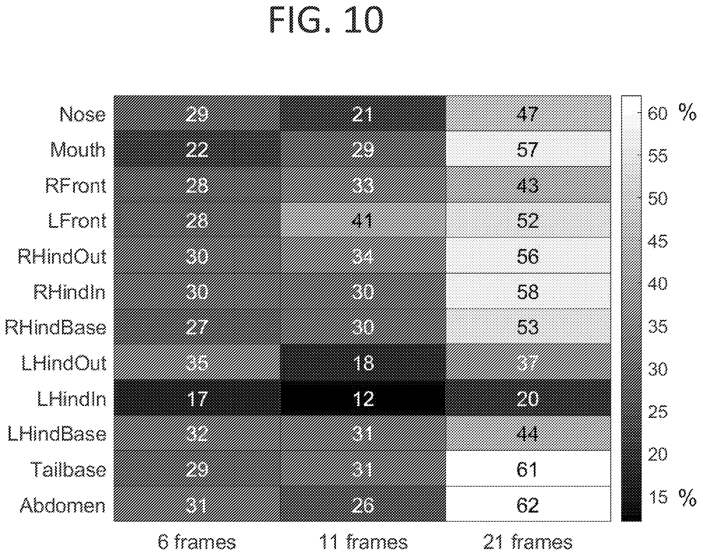

The 12 point GentleBoost model has 1047 inputs but only 385 actually contributed information to the classifier and, each useful input contributed a small amount of information and there were no dominant cues. Each of the 12 body-parts and 3 time windows are included multiple times in the 1047 inputs and the heatmap of FIG. 10 shows the percentage of useful representation as a proportion of all possible opportunities for that variable. The time-window with greatest information was a window of 21 frames (700 ms) with approximately 46% of all used cues in this window. Licking behavior generally extends beyond a second and the 700 ms window appears to be sufficient for capturing the ongoing behavior. FIG. 10 illustrates the relative importance of each window size and each body-part to the model with a heat-map of body-parts and window sizes highlighting the actual contribution of each to the decision of the classifier as a percentage of the possible contribution for each.

Examination of the relative information content of body-part points can be used to determine the most valuable points to keep for a model of this type (see FIG. 10). All points on the right hind paw, mouth and nose appeared to provide high levels of useful information about contact between right hind paw and mouth. Other body points contributed information about body shape and although the base point on the left hind paw is useful as a relative comparison (average 36% use), it may not be necessary to include all three points for the left hind paw (that the formalin is not administered in), as the outer (average 30%) and inner (average 16%) points were the least used. Inclusion of the front paws however appeared to be more useful as the mouse often uses the front paws to hold the hind paw while licking.

### Classifier validation for short videos

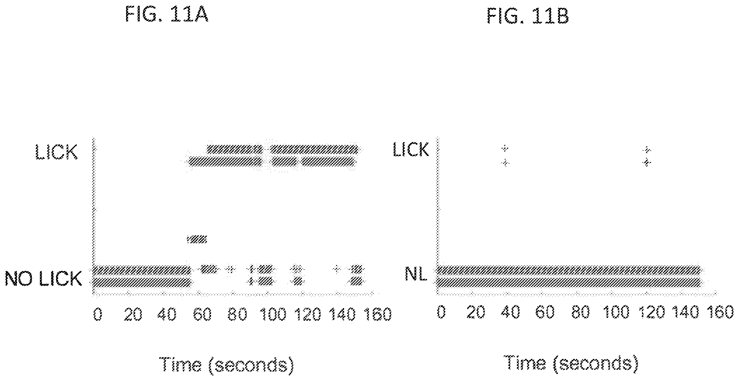

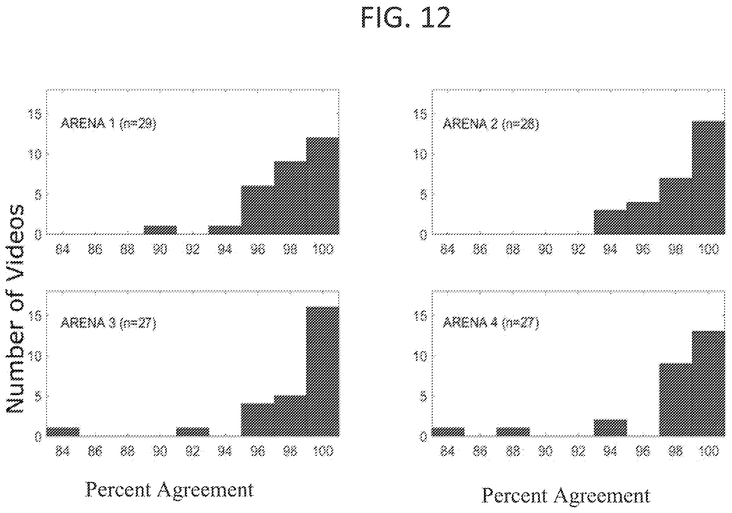

The GentleBoost classifier performance was then tested on 111 new short video clips (from 111 different mice: with 71 completely novel videos and new clips from 40 training videos) for a combined total of about 284 minutes of testing. Each video was manually annotated for licking behavior, for a single mouse in an arena, with temporal resolution of a second. Mice were from all enclosures and an approximately equal number of arenas were annotated (see FIG. 9). FIGS. 11A and 11B show the results of 2 videos with a direct comparison of the human / manual classification of licking behavior in the videos and the automated phenotyping system classification of licking behavior in the videos. The human / manual classification temporal resolution was poorer than the model and therefore a match of licking behavior was recorded if the automated phenotyping system was within +/- 15 frames of the manual classification (i.e., within a second). The percentage of frames in agreement between the automated phenotyping system and the manual classification is shown for all 111 videos in FIG. 12.

Forty-three video clips had no licking behavior and the average agreement over these videos was 98.8% which indicates a low false positive level. FIG. 12 shows that matching on two videos was less accurate with performance in the range of 84% agreement. Close examination of these videos revealed ambiguous behavior and it was difficult for manual classification to ascertain if the mouse was licking or not. For example on one video the mouth was apparently in contact with the right hind paw but it was obscured by the tail, so licking could be marked only by inference and not by manual observation. The other video showed a lot of grooming of the leg and paw area and it was difficult to score purely paw licking. These behaviors were not typical but are hard to classify and different human observers do not agree with each other under these circumstances.

### Inter-observer validation

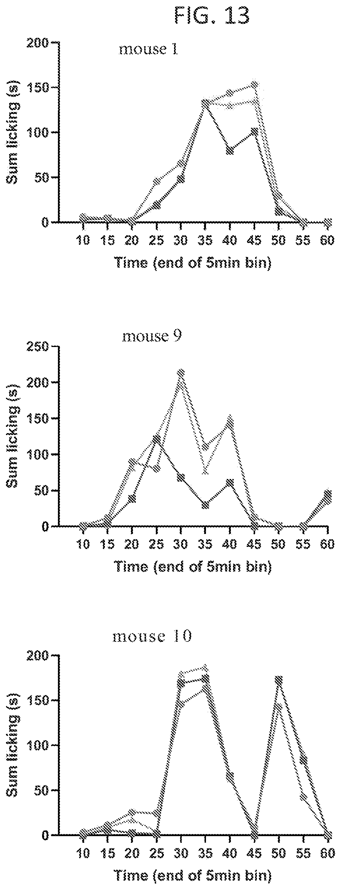

To test human inter-observer reliability, videos (60 minutes) of three mice were annotated by two observers, visualized using Noldus Media Recorder 4 software. The observations were summed in 5 minute intervals and the correlation between observers was generally good (Pearson r = 1.0, 0.82, and 0.97). Both human observers agreed about what constituted licking but disagreed on exactly when to start and stop recording / labeling the behavior. Consequently, licking bouts were sometimes scored as continuous by one observer but as a series of short bouts by the other observer. For observations of mouse 9, these differences in observer scoring resulted in several substantially different measures: for example, two 5 minute bins were recorded as 67 and 60 seconds by Observer 1, and 197 and 151 seconds by Observer 2 (see FIG. 13). Classification by both human observers were compared to the classification by the automated phenotyping system and again agreement was quite high (Observer 1 Pearson r = 0.98, 0.75, 0.95; Observer 2 Pearson r = 0.98, 0.96, 0.99). For mouse 9, the automated phenotyping system appeared to be in better agreement with Observer 2, with the aforementioned 5 minute bins recorded as 213 and 141 seconds of licking (see FIG. 13).

### Strain comparison validation

Manual classification methods have been used for the formalin test to compare the licking response of C57BL/6NCrl compared to C57BL/6J mice in Phase I and Phase II, and found that male C57BL/6NCrl showed a reduced licking response in the Phase II of nociception response (measured as 20-45 minutes) but there was no significant difference for females. To validate the utility of the automated phenotyping system under experimental conditions, the formalin test was conducted comparing similar mouse strains, (Jackson Laboratory: C57BL/6NJ male n = 45, female = 30: C57BL/6J male = 46, female =30). Because the mice in the instant study were anesthetized for the injection, the first five minutes of the nociception response, known as Phase I, were atypical and were not included in the analysis. All of the data was run through the system but the automated phenotyping system determined the start frame for each mouse and then skipped 9000 frames (approximately 5 minutes) before binning the data into 17 five-minute bins (5-10: 85-90) of cumulative licking behavior in seconds.

FIGS. 14-15A-D present graphs comparing licking behavior in male and female mice of strains C57BL/6J and C57BL/6NJ. FIG. 14 shows licking summed in a single bin over the 20-45 minutes post-injection period; mean and SEM are shown separated by sex (significant 2-way ANOVA sex X strain interaction 0.05; * indicates significant post-hoc comparison between strains for each sex). FIG. 15A shows mean and SEM of licking summed in 5 minute bins and displayed for 90 minutes post-injection for females of both strains (bins range from minutes 5-10 to 85-90). FIG. 15B shows mean and SEM of licking summed in 5 minute bins and displayed for 90 minutes post-injection for males of both strains (bins range from minutes 5-10 to 85-90). FIG. 15C shows the percentage from bootstrapping of significant t-tests (alpha = 0.05) showing the difference between the strains by sample size for metrics binned over the periods of 20-45 minutes. FIG. 15D shows the percentage from bootstrapping of significant t-tests (alpha = 0.05) showing the difference between the strains by sample size for metrics binned over the periods of 10-60 minutes.

FIG. 14 shows the summed licking behavior in time bins (20 - 45 minutes post-injection), where male C57BL/6NJ showed reduced licking compared to C57BL/6J male mice. The female mice however showed the reverse pattern with C57BL/6NJ licking more (Sex by Strain interaction F_{(1,147)} = 9.99 p = 0.0019; Holms-Sidak multiple comparisons for male and female p = 0.042). The time course of the responses over the full 90 minutes reveals differences more clearly between the sexes and strains (see FIGS. 15A and 15B). The curves differ in both timing and amplitude of licking, and the choice of how the data is binned for analysis will determine if differences between sexes or strains are detected. FIGS. 15C and 15D compare bootstrapped statistical analysis (alpha level 0.05) for two different bin choices with increasing sample size. Replicating the previously described 20-45 minute bin showed that as sample size increased, the probability of finding a significant difference between the strains also increased, for both males and females (see FIG. 15C). For females, this bin sizing appears to maximize the onset timing difference of the response between strains. The larger bin size of 10-60 minimizes the timing difference for females and bootstrapped comparisons show that an increase of sample size does not alter showing statistical significance (FIG. 15D). The female probability remains near 5% which would be the level due to chance (alpha of 0.05); the females have the same amount of licking. However, the male probability of detecting a difference increases with sample size, the males appear to differ in the amount of licking, in both the amplitude and duration of peak behavior.

One strategy is to use a single summed bin to examine the Phase II period. Although that strategy is likely to be the best choice for revealing a general difference in summed lick duration, it risks losing information about phase differences in the timing of behavior. The choice of bin duration and starting time to analyze Phase II can vary, for example 10-30, 10-60, 10-90, 10-45, 15-45, 20-45, or 20-60. Bootstrapping with different bins indicates that bin choice could contribute to inconsistent results of studies if there is a temporal difference between the strains, sexes or drug treatments of interest. The mice in this experiment were anesthetized which may also have influenced the timing of the behavior as the early part of the response was clearly reduced. The automated phenotyping system showed that C57BL/6N males lick less regardless of the bin choice but bin size for females heavily influenced the outcome. Experiments using either C57BL/6N or C57BL/6N as control mice need to consider the sexes separately as they show clear differences in timing over Phase II. The automated phenotyping system allows experimenters to easily extend the length of the formalin experiment without incurring lengthy video annotation costs. Differences in timing may be evident over the longer duration (60 or 90 minutes) allowing for the possibility of anesthesia effects and for informed choices about bin size.

### Example 2 Devices and Systems

One or more of the trained ML models of the automated phenotyping system may take many forms, including a neural network. A neural network may include a number of layers, from an input layer through an output layer. Each layer is configured to take as input a particular type of data and output another type of data. The output from one layer is taken as the input to the next layer. While values for the input data / output data of a particular layer are not known until a neural network is actually operating during runtime, the data describing the neural network describes the structure, parameters, and operations of the layers of the neural network.

One or more of the middle layers of the neural network may also be known as the hidden layer. Each node of the hidden layer is connected to each node in the input layer and each node in the output layer. In the case where the neural network comprises multiple middle networks, each node in a hidden layer will connect to each node in the next higher layer and next lower layer. Each node of the input layer represents a potential input to the neural network and each node of the output layer represents a potential output of the neural network. Each connection from one node to another node in the next layer may be associated with a weight or score. A neural network may output a single output or a weighted set of possible outputs.

In one aspect, the neural network may be constructed with recurrent connections such that the output of the hidden layer of the network feeds back into the hidden layer again for the next set of inputs. Each node of the input layer connects to each node of the hidden layer. Each node of the hidden layer connects to each node of the output layer. The output of the hidden layer is fed back into the hidden layer for processing of the next set of inputs. A neural network incorporating recurrent connections may be referred to as a recurrent neural network (RNN).

In some embodiments, the neural network may be a long short-term memory (LSTM) network. In some embodiments, the LSTM may be a bidirectional LSTM. The bidirectional LSTM runs inputs from two temporal directions, one from past states to future states and one from future states to past states, where the past state may correspond to characteristics for the video data for a first time frame and the future state may corresponding to characteristics for the video data for a second subsequent time frame.

Processing by a neural network is determined by the learned weights on each node input and the structure of the network. Given a particular input, the neural network determines the output one layer at a time until the output layer of the entire network is calculated.

Connection weights may be initially learned by the neural network during training, where given inputs are associated with known outputs. In a set of training data, a variety of training examples are fed into the network. Each example typically sets the weights of the correct connections from input to output to 1 and gives all connections a weight of 0. As examples in the training data are processed by the neural network, an input may be sent to the network and compared with the associated output to determine how the network performance compares to the target performance. Using a training technique, such as back propagation, the weights of the neural network may be updated to reduce errors made by the neural network when processing the training data.

Various machine learning techniques may be used to train and operate models to perform various steps described herein, such as user recognition feature extraction, encoding, user recognition scoring, user recognition confidence determination, etc. Models may be trained and operated according to various machine learning techniques. Such techniques may include, for example, neural networks (such as deep neural networks and/or recurrent neural networks), inference engines, trained classifiers, etc. Examples of trained classifiers include Support Vector Machines (SVMs), neural networks, decision trees, AdaBoost (short for "Adaptive Boosting") combined with decision trees, and random forests. Focusing on SVM as an example, SVM is a supervised learning model with associated learning algorithms that analyze data and recognize patterns in the data, and which are commonly used for classification and regression analysis. Given a set of training examples, each marked as belonging to one of two categories, an SVM training algorithm builds a model that assigns new examples into one category or the other, making it a non-probabilistic binary linear classifier. More complex SVM models may be built with the training set identifying more than two categories, with the SVM determining which category is most similar to input data. An SVM model may be mapped so that the examples of the separate categories are divided by clear gaps. New examples are then mapped into that same space and predicted to belong to a category based on which side of the gaps they fall on. Classifiers may issue a "score" indicating which category the data most closely matches. The score may provide an indication of how closely the data matches the category.

In order to apply the machine learning techniques, the machine learning processes themselves need to be trained. Training a machine learning component such as, in this case, one of the first or second models, requires establishing a "ground truth" for the training examples. In machine learning, the term "ground truth" refers to the accuracy of a training set's classification for supervised learning techniques. Various techniques may be used to train the models including backpropagation, statistical learning, supervised learning, semi-supervised learning, stochastic learning, or other known techniques.

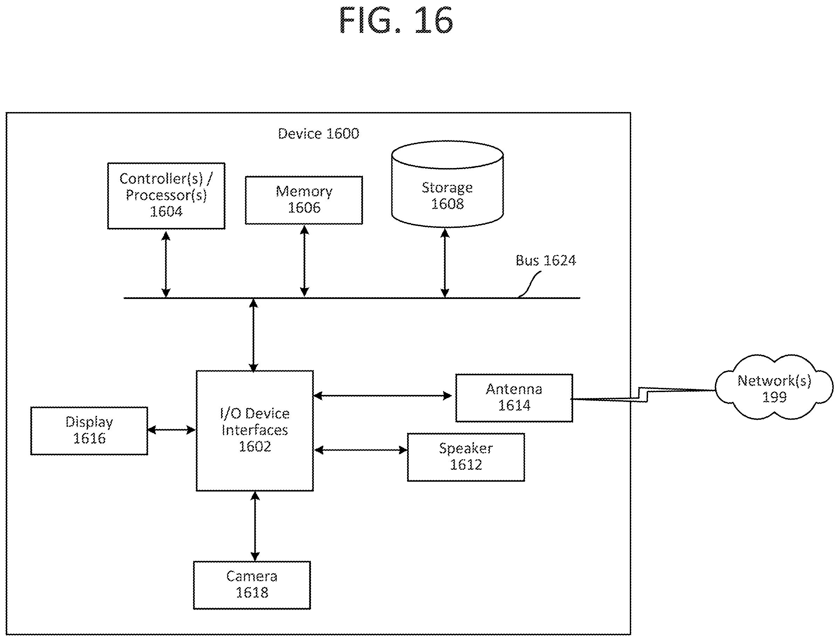

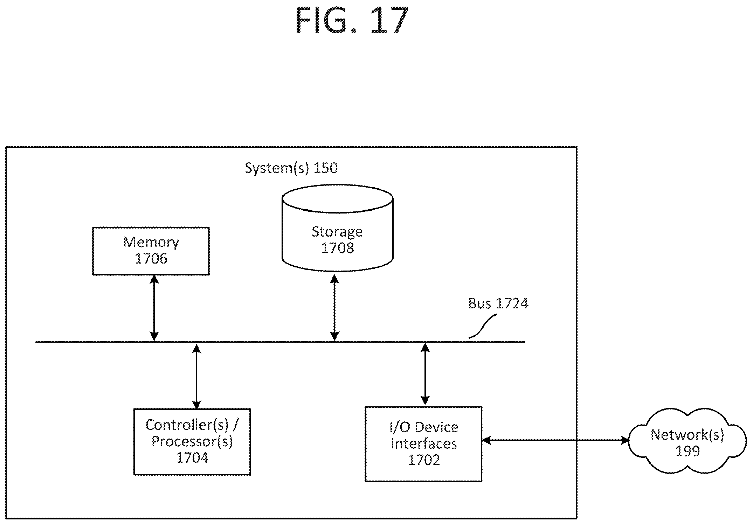

FIG. 16 is a block diagram conceptually illustrating a device 1600 that may be used with the system. FIG. 17 is a block diagram conceptually illustrating example components of a remote device, such as the system(s) 150, which may assist processing of video data, identifying subject behavior, etc. A system(s) 150 may include one or more servers. A "server" as used herein may refer to a traditional server as understood in a server / client computing structure but may also refer to a number of different computing components that may assist with the operations discussed herein. For example, a server may include one or more physical computing components (such as a rack server) that are connected to other devices / components either physically and/or over a network and is capable of performing computing operations. A server may also include one or more virtual machines that emulates a computer system and is run on one or across multiple devices. A server may also include other combinations of hardware, software, firmware, or the like to perform operations discussed herein. The server(s) may be configured to operate using one or more of a client-server model, a computer bureau model, grid computing techniques, fog computing techniques, mainframe techniques, utility computing techniques, a peer-to-peer model, sandbox techniques, or other computing techniques.

Multiple systems 150 may be included in the overall system of the present disclosure, such as one or more systems 150 for performing point / body part tracking, one or more systems 150 for frame-level feature extraction, one or more systems 150 for behavior classification, one or more systems 150 for training / configuring the automated phenotyping system, etc. In operation, each of these systems may include computer-readable and computer-executable instructions that reside on the respective device 150, as will be discussed further below.

Each of these devices (1600/150) may include one or more controllers/processors (1604/1704), which may each include a central processing unit (CPU) for processing data and computer-readable instructions, and a memory (1606/1706) for storing data and instructions of the respective device. The memories (1606/1706) may individually include volatile random access memory (RAM), non-volatile read only memory (ROM), non-volatile magnetoresistive memory (MRAM), and/or other types of memory. Each device (1600/150) may also include a data storage component (1608/1708) for storing data and controller/processor-executable instructions. Each data storage component (1608/1708) may individually include one or more non-volatile storage types such as magnetic storage, optical storage, solid-state storage, etc. Each device (1600/150) may also be connected to removable or external non-volatile memory and/or storage (such as a removable memory card, memory key drive, networked storage, etc.) through respective input/output device interfaces (1602/1702).

Computer instructions for operating each device (1600/150) and its various components may be executed by the respective device's controller(s)/processor(s) (1604/1704), using the memory (1606/1706) as temporary "working" storage at runtime. A device's computer instructions may be stored in a non-transitory manner in non-volatile memory (1606/1706), storage (1608/1708), or an external device(s). Alternatively, some or all of the executable instructions may be embedded in hardware or firmware on the respective device in addition to or instead of software.

Each device (1600/150) includes input/output device interfaces (1602/1702). A variety of components may be connected through the input/output device interfaces (1602/1702), as will be discussed further below. Additionally, each device (1600/150) may include an address/data bus (1624/1724) for conveying data among components of the respective device. Each component within a device (1600/150) may also be directly connected to other components in addition to (or instead of) being connected to other components across the bus (1624/1724).

Referring to FIG. 16, the device 1600 may include input/output device interfaces 1602 that connect to a variety of components such as an audio output component such as a speaker 1612, a wired headset or a wireless headset (not illustrated), or other component capable of outputting audio. The device 1600 may additionally include a display 1616 for displaying content. The device 1600 may further include a camera 1618.

Via antenna(s) 1614, the input/output device interfaces 1602 may connect to one or more networks 199 via a wireless local area network (WLAN) (such as WiFi) radio, Bluetooth, and/or wireless network radio, such as a radio capable of communication with a wireless communication network such as a Long Term Evolution (LTE) network, WiMAX network, 3G network, 4G network, 5G network, etc. A wired connection such as Ethernet may also be supported. Through the network(s) 199, the system may be distributed across a networked environment. The I/O device interface (1602/1702) may also include communication components that allow data to be exchanged between devices such as different physical servers in a collection of servers or other components.

The components of the device(s) 1600 or the system(s) 150 may include their own dedicated processors, memory, and/or storage. Alternatively, one or more of the components of the device(s) 1600, or the system(s) 150 may utilize the I/O interfaces (1602/1702), processor(s) (1604/1704), memory (1606/1706), and/or storage (1608/1708) of the device(s) 1600, or the system(s) 150, respectively.

As noted above, multiple devices may be employed in a single system. **In** such a multi-device system, each of the devices may include different components for performing different aspects of the system's processing. The multiple devices may include overlapping components. The components of the device 1600, and the system(s) 150, as described herein, are illustrative, and may be located as a stand-alone device or may be included, in whole or in part, as a component of a larger device or system.

The concepts disclosed herein may be applied within a number of different devices and computer systems, including, for example, general-purpose computing systems, video / image processing systems, and distributed computing environments.

The above aspects of the present disclosure are meant to be illustrative. They were chosen to explain the principles and application of the disclosure and are not intended to be exhaustive or to limit the disclosure. Many modifications and variations of the disclosed aspects may be apparent to those of skill in the art. Persons having ordinary skill in the field of computers and speech processing should recognize that components and process steps described herein may be interchangeable with other components or steps, or combinations of components or steps, and still achieve the benefits and advantages of the present disclosure. Moreover, it should be apparent to one skilled in the art, that the disclosure may be practiced without some or all of the specific details and steps disclosed herein.

Aspects of the disclosed system may be implemented as a computer method or as an article of manufacture such as a memory device or non-transitory computer readable storage medium. The computer readable storage medium may be readable by a computer and may comprise instructions for causing a computer or other device to perform processes described in the present disclosure. The computer readable storage medium may be implemented by a volatile computer memory, non-volatile computer memory, hard drive, solid-state memory, flash drive, removable disk, and/or other media. In addition, components of system may be implemented as in firmware or hardware.

### Example 3. Automated licking measurement; composite nociceptive index; testing genetic variation in nociceptive response

Measuring licking in the widely used open field arena with a top-down camera view was automated. Additionally, measuring a variety of possible nocifensive behaviors was automated for the purpose of developing a composite nociceptive index.

### Methods

Methods used were as described in Example 1, except as otherwise described below herein.

### Open field video data collection, per-frame measures, and features

Top-down video data 104 of each mouse in a one hour open field session was collected as described above herein in Example 1 and as previously described (FIG. 18A) [Kumar, V. et al. PNAS 108, 15557-15564. ISSN: 0027-8424 (2011); Geuther, B. et al., Communications Biology 2, 124 (Mar. 2019)]. Open field video was processed by a deep neural network based pose estimation network and a tracking network to produce a 12-point pose skeleton and an ellipse fit track of the mouse for each frame [Sheppard, K. et al. bioRxiv. doi.org/10.11:1/2020.12.29.424780 (2020); Geuther, B. et al., Communications Biology 2, 124 (Mar. 2019)]. These per-frame measures were used to make behavior classifiers, and were also used to engineer features such as traditional open field measures of anxiety, hyperactivity [Geuther, B. et al., Communications Biology 2, 124 (Mar. 2019)], neural network-based grooming [Geuther, B. Q. et al. Elife 10, e63207 (2021)], and novel gait measures [Sheppard, K. et al. bioRxiv. doi.org/10.1101/2020. 12.29.424780 (2020)]. Figure 22 provides information for video features used in certain embodiments of the invention.

### Results

Genetic variation in nociceptive response was also of interest. Variation in licking behavior in inbred mouse strains in response to formalin had previously been described [Mogil, J. et al. Pain 80, 67-82 (1999)]. Strains were selected ranging from higher licking responders (C57BL6J and C3HHeJ) to lower licking responders (BALBcJ and AJ) [Mogil, J. et al. Pain 80, 67-82 (1999)]. Both male and female mice from each strain were used because sex differences in response had also been described. Four doses of formalin were used (0.00%, 1.25%, 2.50%, and 5.00%), with each dosage being tested on at least five male and five female mice for each strain, resulting in a dataset of 194 mice (FIG. 18A).

One behavior classified using JABS was licking/biting. Licking is considered the most important nocifensive behavior to quantify in formalin assays [Saddi, G.-M. and Abbott, F., Pain 89, 53-63 (2001); Abbot, F. et al., Pain, 83, 561-569 (1999); Wotton, J. M. et al., Molecular Pain 16, 1744806920958596 (2020)]. The licking classifier was trained on a set of videos from a prior experiment using male and female C57BL6/J mice at doses of 0.00% (saline), 0.27%, 0.87%, and 2.5% formalin. A behaviorist densely labeled one-hour videos from each of four strains at 5.00% formalin for a total of four hours of video. The classifier was used on those four videos and high frame agreement was found for all videos. FIG. 18B shows agreement at the frame level. To further investigate the agreement between the labeler and the classifier, the overlap between bouts of licking found by the classifier and the labeler were compared. For each bout found by either the classifier or the labeler, the amount of frames within that bout where both the classifier and labeler agreed that licking was happening was calculated. If at least half of the frames were in agreement, that bout was considered to be overlapping. FIG. 18C shows the percentage of overlapping bouts in which the bout was greater than one second. Using the frame level classification and labels, the number of licking bouts, time spent licking, and average length of licking bout were calculated for the videos (FIG. 18D-18F). These measures were compared between the classifier and the labeler and showed that while for most videos the classifier was slightly more conservative than the labeler, the amount of licking found overall was comparable. Combined, these results showed that this was a reasonable licking classifier.

Next, per-video licking measures were classified and calculated for the dataset. When examining the differences in time spent licking across dosages and across strains for male and female mice, clear differences were observed across strain and sex (FIG. 18G-18H). C57BL6/J mice showed high licking, as previously described [Mogil, J. et al. Pain 80, 67-82 (1999)], C3HHeJ mice showed a medium amount of licking, and BALBcJ mice and AJ mice showed low licking. Though licking was a good measure to show dose-dependent nociception in certain strains of mice, it may not be as reliable for low responders like BALBcJ and AJ mice. Because composite nociception score measuring multiple behaviors has been found to be a more robust method of quantifying nociception in the formalin assay, multiple automated measures were investigated.

Paw shaking is another known nocifensive behavior in the formalin assay. A classifier for shaking behavior was trained. Time spent shaking across dosages for males and females is shown in FIG. 19A and FIG. 19B, respectively. Next, it was hypothesized that distressed mice may rear less often, and classifier was trained for rearing supported by a wall. Time spent rearing for males and females is shown in FIG. 19C and FIG. 19D, respectively; accuracy measures from ten-fold cross validation are shown in FIG. 19E. It was found that mice given high doses of formalin tended to have bouts of freezing, staying very still for several seconds at a time. Freezing bouts were heuristically determined by taking the average speed of the nose, base of head, and base of tail points at each frame, and finding periods of at least three seconds where the average speed of the mouse was close to zero. From those measurements, a variety of features were calculated. Time spent in freezing bouts between three to six seconds long for male and female mice across dosages is shown in FIG. 19F and FIG. 19G, respectively. Interestingly, BALBcJ mice and AJ mice were higher responders than C57BL6/J mice with respect to freezing bouts. Gait was also examined using previously described methods to extract stride measures from freely moving mice in the open field [Sheppard, K. et al. bioRxiv. doi.org/10.1101/2020.12.29.424780 (2020)]. For many BALBcJ mice and AJ mice, few to no strides were found throughout the video. Both strains are known for their low locomotor activity. Thus, nearly all measures related to movement in the open field had non-significant correlation with formalin dosage for BALBcJ mice and AJ mice (FIG. 20). Examining correlations across all measures, clear strain differences between the high responders and low responders were observed (FIG. 20).

Next, a cumulative (logit) link model [Agresti, A. Categorical data analysis (John Wiley & Sons, 2003)] was fitted to the ordinal response (Dose) using features in FIG. 22. The feature weights/coefficients (*β*) extracted from the model were used to construct a univariate pain scale. Next, the data with ordinal class labels (Dose) were projected onto the univariate pain scale axis (FIG. 21A). The vertical dashed line corresponding to the intercept from the cumulative link model that separates Dose levels 1 and 2 (FIG. 21A) was plotted and was used to separate animals belonging to no/low pain (Dose levels 0,1) and high pain (Dose levels 2,3) groups below in binary classification analysis below herein (FIG. 21C and 21D). The contributions of individual features to the univariate pain scale were obtained using feature coefficients/weights (*β*) (FIG. 21B). A binary logistic regression model was built using different sets of features ['Open field' (solid gray dot), 'Other' include engineered features and features obtained from behavior classifier ("X"), and 'All' include both open field and other ("*")]. Next, the accuracy metric of the classifier obtained using leave one animal out cross-validation was used to assess the efficacy of the pain scale in classifying animals into low (0,1 Dose levels) and high (2,3 Dose levels) pain groups (FIG. 21C). It was found that including 'All' features ("*") gave marginally better accuracy for classifying animals belonging to low and high pain groups compared to including only the 'Other' set of features ("X"). A similar procedure as with FIG. 21C was used, but the classifier was trained on animals belonging to all strains but one strain (leave one strain out cross-validation). Performances of different sets of features (similar to FIG. 21C) for classifying animals (belonging to the left out strain) to their respective pain groups (FIG. 21D) were compared to assess differences due to strains. As before, the 'All' feature set ("*") offered marginal improvement in predicting the pain class over the "Other" set of features. In fact, for C57BL/6NJ the 'Other' set of features outperformed the "All" set of features. Another interesting finding was that open field metrics (solid gray dot) had different predictive accuracy for different strains. For example, up to 60% accuracy was obtained with open field metrics for classifying C57BL/6NJ animals into low/high pain groups (FIG. 21D). In contrast, open field metrics were unreliable for predicting pain class in AJ animals.

### Equivalents

Although several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto; the invention may be practiced otherwise than as specifically described and claimed. The present invention is directed to each individual feature, system, article, material, and/or method described herein. In addition, any combination of two or more such features, systems, articles, materials, and/or methods, if such features, systems, articles, materials, and/or methods are not mutually inconsistent, is included within the scope of the present invention. All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements, and/or steps are in any way required for one or more embodiments or that one or more embodiments necessarily include logic for deciding, with or without other input or prompting, whether these features, elements, and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

All references, patents and patent applications and publications that are cited or referred to in this application are incorporated by reference in their entirety herein.

The present invention provides, inter alia, the subject matter of the following clauses:
1. A computer-implemented method comprising: receiving video data representing a video capturing movements of a subject; determining, using the video data, first point data identifying a location of a first body part of the subject for a first frame during a first time period; determining, using the video data, second point data identifying a location of a second body part of the subject for the first frame; determining, using the first point data and the second point data, first distance data representing a distance between the first body part and the second body part; determining a first feature vector corresponding to at least the first frame and a second frame, the first feature vector including at least the first distance data and second distance data; processing, using a trained model, at least the first feature vector, the trained model configured to identify a likelihood of the subject exhibiting a behavior during the first time period; and determining, based on the processing of at least the first feature vector, a first label corresponding to the first time period, the first label identifying a first behavior of the subject during the first time period.
2. The computer-implemented method of clause 1, further comprising: determining, using the video data, third point data identifying a location of a third body part of the subject for the first frame.
3. The computer-implemented method of clause 2, further comprising: determining, using the first point data and the third point data, second distance data representing a distance between the first body part and the third body part; determining a second feature vector corresponding to the first frame to include at least the second distance data; and wherein processing using the trained model comprises processing the first feature vector and the second feature vector using the trained model.
4. The computer-implemented method of clause 2, further comprising: determining, using the first point data, the second point data and the third point data, first angle data representing an angle corresponding to the first body part, the second body part and the third body part; determining a second feature vector corresponding to at least the first frame, the second feature vector including at least the first angle data; and wherein processing using the trained model further comprises processing the first feature vector and the second feature vector using the trained model.
5. The computer-implemented method of clause 4, further comprising: determining, using the video data, fourth point data identifying a location of the first body part for a second frame during the first time period; determining, using the video data, fifth point data identifying a location of the second body part for the second frame; determining, using the video data, sixth point data identifying a location of the third body part for the second frame; determining, using the fourth point data and the fifth point data, third distance data representing a distance between the first body part and the second body part for the second frame; determining, using the fourth point data and the sixth point data, fourth distance data representing a distance between the first body part and the third body part for the second frame; determining, using the fourth point data, the fifth point data and the sixth point data, second angle data representing an angle corresponding to the first body part, the second body part and the third body part for the second frame; and determining the second feature vector to include at least the third distance data, the fourth distance data, and the second angle data.
6. The computer-implemented method of clause 1, wherein the second distance data represents a distance between the first body part and the second body part for the second frame during the first time period.
7. The computer-implemented method of clause 6, further comprising: calculating metric data corresponding to the first frame using at least the first distance data and the second distance data, wherein the first feature vector includes the metric data.
8. The computer-implemented method of clause 7, wherein the metric data represents statistical analysis corresponding to at least the first distance data and the second distance data, the statistical analysis being at least one of a mean, a standard deviation, a median, and a median absolute deviation.
9. The computer-implemented method of clause 1, further comprising: processing the video data using an additional trained model to determine the first point data, wherein the first point data includes pixel data representing the location of the first body part.
10. The computer-implemented method of clause 1, further comprising: processing the video data using an additional trained model to determine a likelihood that a pixel coordinate corresponds to the first body part; and determining the first point data based at least in part on the likelihood satisfying a threshold, the first point data including the pixel coordinate.
11. The computer-implemented method of clause 1, further comprising: determining, using the video data, additional point data identifying locations of at least 12 portions of the subject for the first frame, wherein the 12 portions includes at least the first body part and the second body part.
12. The computer-implemented method of clause 11, further comprising: determining additional distance data representing distances between a plurality of body portion-pairs for the first frame, the plurality of body portion-pairs formed using pairs of the 12 portions of the subject, and wherein the first feature vector includes the additional distance data.
13. The computer-implemented method of clause 11, further comprising: determining additional angle data representing angles corresponding to a plurality of body-portion trios for the first frame, the plurality of body portion-trios formed by selecting three of the 12 portions of the subject, and wherein the first feature vector includes the additional angle data.
14. The computer-implemented method of clause 1, further comprising: determining additional feature vectors corresponding to six frames during the first time period, the six frames including at least the first frame and the second frame; calculating metric data using the additional feature vectors, the metric data representing at least one of a mean, a standard deviation, a median, and a median absolute deviation; and processing the metric data using the trained model to determine the first label.
15. The computer-implemented method of clause 14, further comprising: determining location data representing pixel coordinates of 12 portions of the subject for the first frame, the location data including at least the first point data, the second point data and the third point data, and wherein processing the metric data using the trained model further includes processing the location data using the trained model.
16. The computer-implemented method of clause 1, further comprising: determining additional feature vectors corresponding to 11 frames during the first time period, the 11 frames including at least the first frame and the second frame; calculating metric data using the additional feature vectors, the metric data representing at least one of a mean, a standard deviation, a median, and a median absolute deviation; and processing the metric data using the trained model to determine the first label.
17. The computer-implemented method of clause 16, wherein the 11 frames includes five frames prior to the first frame and five frames after the first frame.
18. The computer-implemented method of clause 1, further comprising: determining additional feature vectors corresponding to 21 frames during the first time period, the 21 frames including at least the first frame and the second frame; calculating metric data using the additional feature vectors, the metric data representing at least one of a mean, a standard deviation, a median, and a median absolute deviation; and processing the metric data using the trained model to determine the first label.
19. The computer-implemented method of clause 18, wherein the 21 frames includes 11 frames prior to the first frame and 11 frames after the first frame.
20. The computer-implemented method of clause 1, wherein the video data represents video capturing movements of more than one subject.
21. The computer-implemented method of clause 1, wherein the trained model is a classifier configured to process feature data corresponding to video frames to determine a behavior exhibited by the subject represented in the video frames, the feature data corresponding to portions of the subject.
22. The computer-implemented method of clause 1, wherein: the first body part is a mouth of the subject; the second body part is right hind foot of the subject; the trained model is configured to identify a likelihood of the subject exhibiting contact between the first body part and the second body part; and the first label indicates the first frame represents contact between the first body part and the second body part.
23. The computer-implemented method of clause 1, wherein the first frame corresponds to 30 milliseconds of video data.
24. The computer-implemented method of clause 1, wherein the video data corresponds to a first video capturing a top view of the subject and a second video capturing a side view of the subject.
25. The computer-implemented method of clause 1, wherein the subject is a mammal.
26. The computer-implemented method of clause 1, wherein the subject is a rodent.
27. The computer-implemented method of clause 1, wherein the subject is a primate.
28. A method of determining a nociceptive behavior in a test subject, the method comprising monitoring a response of the test subject, wherein a means of the monitoring comprises a computer-implemented method of clause 1.
29. The method of clause 28, wherein the test subject has a pain condition.
30. The method of clause 29, wherein the pain condition comprises one of more of: inflammatory pain, neuropathic pain, muscle pain, joint pain, chronic pain, visceral pain, cancer pain, and postoperative pain.
31. The method of clause 28, wherein the test subject is an animal model of a pain condition.
32. The method of clause 28, wherein a pain is induced in the test subject.
33. The method of clause 32, wherein inducing the pain in the test subject comprises inducing inflammation in the test subject.
34. The method of clause 33, wherein inducing inflammation comprises contacting the test subject with one or more of: heat, light, pressure, cold, and a chemical agent.
35. The method of clause 34, wherein the chemical agent comprises one or more of formalin and acetone.
36. The method of clause 34 or 35, wherein inducing the pain comprises one or more of contacting the test subject with the chemical agent and injecting the test subject with the chemical agent.
37. The method of clause 28, wherein the test subject is a genetically engineered test subject.
38. The method of clause 28, wherein the test subject is a rodent, and optionally is a mouse.
39. The method of clause 38, wherein the mouse is a genetically engineered mouse.
40. The method of clause 28, further comprising administering a candidate therapeutic agent to the test subject.
41. The method of clause 40, wherein if pain is induced in the test subject, the candidate therapeutic agent is administered to the test subject prior to inducing the pain in the test subject.
42. The method of clause 40, wherein if pain is induced in the test subject, the candidate therapeutic agent is administered to the test subject after inducing the pain in the test subject.
43. The method of clause 28, wherein a result of the monitoring of the test subject is compared to a control result.
44. The method of clause 43, wherein the control result is a result from a control subject monitored with the computer-implemented method.
45. The method of clause 44, wherein pain is induced in the control subject.
46. The method of clause 44, wherein the control subject is an animal model of the pain condition.
47. The method of clause 45 or 46, wherein the control subject is not administered the candidate therapeutic agent.
48. The method of clause 44, wherein the control subject is administered a dose of the candidate therapeutic agent that is different than the dose of the candidate therapeutic agent administered to the test subject.
49. The method of clause 44, wherein the control result is a result from a previous monitoring of the test subject with the computer-implemented method.
50. The method of clause 28, wherein the monitoring of the subject identifies a chronic pain condition in the subject.
51. The method of clause 28, wherein the monitoring of the subject identifies efficacy of a candidate therapeutic agent to treat a pain condition.
52. A method of identifying efficacy of a candidate therapeutic agent to treat a pain condition in a subject, comprising: administering to a test subject the candidate therapeutic agent and monitoring the test subject, wherein a means of the monitoring comprises a computer- implemented method of clause 1, and wherein results of the monitoring indicating reduced pain in the test subject identifies an efficacy of the candidate therapeutic agent to treat the pain condition.
53. The method of clause 52, wherein the pain condition comprises one of more of: inflammatory pain, neuropathic pain, muscle pain, joint pain, chronic pain, visceral pain, cancer pain, and postoperative pain.
54. The method of clause 52, wherein the test subject has the pain condition.
55. The method of clause 52, wherein the test subject is an animal model of the pain condition.
56. The method of clause 52, wherein a pain is induced in the test subject prior to the monitoring.
57. The method of clause 52, wherein inducing the pain in the test subject comprises inducing inflammation in the test subject.
58. The method of clause 57, wherein inducing inflammation comprises contacting the test subject with one or more of: heat, light, pressure, cold, and a chemical agent.
59. The method of clause 58, wherein the chemical agent comprises one or more of formalin and acetone, and wherein inducing the pain comprises one or more of contacting the test subject with the chemical agent and injecting the test subject with the chemical agent.
60. The method of clause 52, wherein the test subject is a genetically engineered test subject.
61. The method of clause 52, wherein the test subject is a rodent, and optionally is a mouse.
62. The method of clause 61, wherein the mouse is a genetically engineered mouse.
63. The method of clause 56, wherein the candidate therapeutic agent is administered to the test subject prior to inducing the pain in the test subject.
64. The method of clause 56, wherein the candidate therapeutic agent is administered to the test subject after inducing the pain in the test subject.
65. The method of clause 52, wherein a result of the monitoring of the test subject is compared to a control result.
66. The method of clause 65, wherein the control result is a result from a control subject monitored with the computer-implemented method.
67. The method of clause 66, wherein pain is induced in the control subject.
68. The method of clause 66, wherein the control subject has the pain condition, and optionally is an animal model of the pain condition.
69. The method of clause 67 or 68, wherein the control subject is not administered the candidate therapeutic agent.
70. The method of clause 66, wherein the control subject is administered a dose of the candidate therapeutic agent that is different than the dose of the candidate therapeutic agent administered to the test subject.
71. The method of clause 66, wherein the control result is a result from a previous monitoring of the test subject with the computer-implemented method.
72. The method of clause 52, further comprising additionally testing the efficacy of the candidate therapeutic agent.
73. A system comprising: at least one processor; and at least one memory comprising instructions that, when executed by the at least one processor, cause the system to: receive video data representing a video capturing movements of a subject; determine, using the video data, first point data identifying a location of a first body part of the subject for a first frame during a first time period; determine, using the video data, second point data identifying a location of a second body part of the subject for the first frame; determine, using the first point data and the second point data, first distance data representing a distance between the first body part and the second body part; determine a first feature vector corresponding to at least the first frame and a second frame, the first feature vector including at least the first distance data and second distance data; process, using a trained model, at least the first feature vector, the trained model configured to identify a likelihood of the subject exhibiting a behavior during the first time period; and determine, based on the processing of at least the first feature vector, a first label corresponding to the first time period, the first label identifying a first behavior of the subject during the first time period.
74. The system of clause 73, wherein the at least one memory further comprises instructions that, when executed by the at least one processor, further cause the system to: determine, using the video data, third point data identifying a location of a third body part of the subject for the first frame.
75. The system of clause 74, wherein the at least one memory further comprises instructions that, when executed by the at least one processor, further cause the system to: determine, using the first point data and the third point data, second distance data representing a distance between the first body part and the third body part; determine a second feature vector corresponding to the first frame to include at least the second distance data; and wherein the instructions that cause the system to process using the trained model further causes the system to process the first feature vector and the second feature vector using the trained model.
76. The system of clause 74, wherein the at least one memory further comprises instructions that, when executed by the at least one processor, further cause the system to: determine, using the first point data, the second point data and the third point data, first angle data representing an angle corresponding to the first body part, the second body part and the third body part; determine a second feature vector corresponding to at least the first frame, the second feature vector including at least the first angle data; and wherein the instructions that cause the system to process using the trained model further causes the system to process the first feature vector and the second feature vector using the trained model.
77. The system of clause 76, wherein the at least one memory further comprises instructions that, when executed by the at least one processor, further cause the system to: determine, using the video data, fourth point data identifying a location of the first body part for a second frame during the first time period; determine, using the video data, fifth point data identifying a location of the second body part for the second frame; determine, using the video data, sixth point data identifying a location of the third body part for the second frame; determine, using the fourth point data and the fifth point data, third distance data representing a distance between the first body part and the second body part for the second frame; determine, using the fourth point data and the sixth point data, fourth distance data representing a distance between the first body part and the third body part for the second frame; determine, using the fourth point data, the fifth point data and the sixth point data, second angle data representing an angle corresponding to the first body part, the second body part and the third body part for the second frame; and determine the second feature vector to include at least the third distance data, the fourth distance data, and the second angle data.
78. The system of clause 73, wherein the second distance data represents a distance between the first body part and the second body part for the second frame during the first time period.
79. The system of clause 78, wherein the at least one memory further comprises instructions that, when executed by the at least one processor, further cause the system to: calculate metric data corresponding to the first frame using at least the first distance data and the second distance data, wherein the first feature vector includes the metric data.
80. The system of clause 79, wherein the metric data represents statistical analysis corresponding to at least the first distance data and the second distance data, the statistical analysis being at least one of a mean, a standard deviation, a median, and a median absolute deviation.
81. The system of clause 73, wherein the at least one memory further comprises instructions that, when executed by the at least one processor, further cause the system to: process the video data using an additional trained model to determine the first point data, wherein the first point data includes pixel data representing the location of the first body part.
82. The system of clause 73, wherein the at least one memory further comprises instructions that, when executed by the at least one processor, further cause the system to: process the video data using an additional trained model to determine a likelihood that a pixel coordinate corresponds to the first body part; and determine the first point data based at least in part on the likelihood satisfying a threshold, the first point data including the pixel coordinate.
83. The system of clause 73, wherein the at least one memory further comprises instructions that, when executed by the at least one processor, further cause the system to: determine, using the video data, additional point data identifying locations of at least 12 portions of the subject for the first frame, wherein the 12 portions includes at least the first body part and the second body part.
84. The system of clause 83, wherein the at least one memory further comprises instructions that, when executed by the at least one processor, further cause the system to: determine additional distance data representing distances between a plurality of body portion-pairs for the first frame, the plurality of body portion-pairs formed using pairs of the 12 portions of the subject, and wherein the first feature vector includes the additional distance data.
85. The system of clause 83, wherein the at least one memory further comprises instructions that, when executed by the at least one processor, further cause the system to: determine additional angle data representing angles corresponding to a plurality of body-portion trios for the first frame, the plurality of body portion-trios formed by selecting three of the 12 portions of the subject, and wherein the first feature vector includes the additional angle data.
86. The system of clause 73, wherein the at least one memory further comprises instructions that, when executed by the at least one processor, further cause the system to: determine additional feature vectors corresponding to six frames during the first time period, the six frames including at least the first frame and the second frame; calculate metric data using the additional feature vectors, the metric data representing at least one of a mean, a standard deviation, a median, and a median absolute deviation; and process the metric data using the trained model to determine the first label.
87. The system of clause 86, wherein the at least one memory further comprises instructions that, when executed by the at least one processor, further cause the system to: determine location data representing pixel coordinates of 12 portions of the subject for the first frame, the location data including at least the first point data, the second point data and the third point data, and wherein the instructions that cause the system to process the metric data using the trained model further causes the system to process the location data using the trained model.
88. The system of clause 73, wherein the at least one memory further comprises instructions that, when executed by the at least one processor, further cause the system to: determine additional feature vectors corresponding to 11 frames during the first time period, the 11 frames including at least the first frame and the second frame; calculate metric data using the additional feature vectors, the metric data representing at least one of a mean, a standard deviation, a median, and a median absolute deviation; and process the metric data using the trained model to determine the first label.
89. The system of clause 88, wherein the 11 frames includes five frames prior to the first frame and five frames after the first frame.
90. The system of clause 73, wherein the at least one memory further comprises instructions that, when executed by the at least one processor, further cause the system to: determine additional feature vectors corresponding to 21 frames during the first time period, the 21 frames including at least the first frame and the second frame; calculate metric data using the additional feature vectors, the metric data representing at least one of a mean, a standard deviation, a median, and a median absolute deviation; and process the metric data using the trained model to determine the first label.
91. The system of clause 90, wherein the 21 frames includes 11 frames prior to the first frame and 11 frames after the first frame.
92. The system of clause 73, wherein the video data represents video capturing movements of more than one subject.
93. The system of clause 73, wherein the trained model is a classifier configured to process feature data corresponding to video frames to determine a behavior exhibited by the subject represented in the video frames, the feature data corresponding to portions of the subject.
94. The system of clause 73, wherein: the first body part is a mouth of the subject; the second body part is right hind foot of the subject; the trained model is configured to identify a likelihood of the subject exhibiting contact between the first body part and the second body part; and the first label indicates the first frame represents contact between the first body part and the second body part.
95. The system of clause 73, wherein the first frame corresponds to 30 milliseconds of video data.
96. The system of clause 73, wherein the video data corresponds to a first video capturing a top view of the subject and a second video capturing a side view of the subject.
97. The system of clause 73, wherein the subject is a mammal.
98. The system of clause 73, wherein the subject is a rodent.
99. The system of clause 73, wherein the subject is a primate.
100. One or more non-transitory computer-readable media comprising computer executable instructions that, when executed, cause at least one processor to perform actions comprising: receiving video data representing a video capturing movements of a subject; determining, using the video data, first point data identifying a location of a first body part of the subject for a first frame during a first time period; determining, using the video data, second point data identifying a location of a second body part of the subject for the first frame; determining, using the first point data and the second point data, first distance data representing a distance between the first body part and the second body part; determining a first feature vector corresponding to at least the first frame and a second frame, the first feature vector including at least the first distance data and second distance data; processing, using a trained model, at least the first feature vector, the trained model configured to identify a likelihood of the subject exhibiting a behavior during the first time period; and determining, based on the processing of at least the first feature vector, a first label corresponding to the first time period, the first label identifying a first behavior of the subject during the first time period.

## Claims

1. A method of determining a nociceptive behavior in a test subject, the method comprising:
monitoring a response of the test subject, wherein monitoring comprises:
receiving video data representing a video capturing movements of a subject;
determining, using the video data, first point data identifying a location of a first body part of the subject for a first frame during a first time period;
determining, using the video data, second point data identifying a location of a second body part of the subject for the first frame;
determining, using the first point data and the second point data, first distance data representing a distance between the first body part and the second body part, wherein the distance between the first body part and the second body part is a first distance frame feature in the first frame;
determining a first feature vector corresponding to at least the first frame and a second frame, the first feature vector including at least the first distance data and second distance data;
processing, using a trained model, at least the first feature vector, the trained model configured to identify a likelihood of the subject exhibiting a behavior during the first time period; and
determining, based on the processing of at least the first feature vector, a first label corresponding to the first time period, the first label identifying a first behavior of the subject during the first time period.

2. The method of claim 1, wherein the test subject has a pain condition comprising one of more of: inflammatory pain, neuropathic pain, muscle pain, joint pain, chronic pain, visceral pain, cancer pain, and postoperative pain.

3. The method of claim 1, wherein the test subject is an animal model of a pain condition.

4. The method of claim 1, further comprising inducing pain in the subject, and wherein inducing the pain in the test subject comprises inducing inflammation in the test subject.

5. The method of claim 4, wherein inducing inflammation comprises contacting the test subject with one or more of: heat, light, pressure, cold, and a chemical agent.

6. The method of claim 5, wherein the chemical agent comprises one or more of formalin and acetone.

7. The method of claim 6, wherein inducing the pain comprises one or more of contacting the test subject with the chemical agent and injecting the test subject with the chemical agent.

8. The method of claim 1, wherein the test subject is:
a) a genetically engineered test subject; or
b) a rodent.

9. The method of claim 1, further comprising administering a candidate therapeutic agent to the test subject.

10. The method of claim 9, wherein if pain is induced in the test subject, the candidate therapeutic agent is administered to the test subject:
i) prior to inducing the pain in the test subject; or
ii) after inducing the pain in the test subject.

11. The method of claim 1, wherein the result of the monitoring of the test subject is compared to a control result, wherein the control result is a result from a control subject.

12. The method of claim 11, wherein:
a) pain is induced in the control subject; or
b) the control subject is an animal model of a pain condition.

13. The method of claim 12, wherein the control subject is not administered a candidate therapeutic agent.

14. The method of claim 13, wherein the control subject:
i) is administered a dose of a candidate therapeutic agent that is different than a dose of the candidate therapeutic agent administered to the test subject; or
ii) is a result from a previous monitoring of the test subject.

15. The method of claim 1, wherein the monitoring of the subject identifies:
a) a chronic pain condition in the subject; or
b) efficacy of a candidate therapeutic agent to treat a pain condition.
